(19) Europäisches Patentamt / European Patent Office / Office européen des brevets

(11) **EP 2 552 397 B1**

(12) **FASCICULE DE BREVET EUROPEEN**

(45) Date de publication et mention de la délivrance du brevet:
**14.12.2016 Bulletin 2016/50**

(21) Numéro de dépôt: **11720140.0**

(22) Date de dépôt: **01.04.2011**

(51) Int Cl.:
*A61K 8/60* (2006.01)　*A61K 8/67* (2006.01)
*A61K 31/07* (2006.01)　*A61K 31/11* (2006.01)
*A61K 31/192* (2006.01)　*A61K 31/203* (2006.01)
*A61K 31/232* (2006.01)　*A61K 31/7008* (2006.01)
*A61Q 19/00* (2006.01)　*A61Q 19/08* (2006.01)
*A61P 17/06* (2006.01)　*A61P 17/16* (2006.01)
*A61P 19/02* (2006.01)　*A61P 29/00* (2006.01)

(86) Numéro de dépôt international:
**PCT/FR2011/050732**

(87) Numéro de publication internationale:
**WO 2011/121250 (06.10.2011 Gazette 2011/40)**

(54) **COMPOSITION COSMETIQUE ET PHARMACEUTIQUE COMPRENANT DE LA N-ACETYL-GLUCOSAMINE-6-PHOSPHATE ET UN COMPOSÉ DE LA FAMILLE DE LA VITAMINE A**

KOSMETISCHE UND PHARMAZEUTISCHE ZUSAMMENSETZUNG MIT N-ACETYLGLUCOSAMIN-6-PHOSPHAT UND EINE VITAMIN-A-VERBINDUNG

COSMETIC AND PHARMACEUTICAL COMPOSITION COMPRISING N-ACETYLGLUCOSAMINE-6-PHOSPHATE AND A VITAMIN A COMPOUND

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.04.2010 FR 1052536**

(43) Date de publication de la demande:
**06.02.2013 Bulletin 2013/06**

(73) Titulaire: **Libragen**
**31400 Toulouse (FR)**

(72) Inventeurs:
• **AURIOL, Daniel**
**F-31300 Toulouse (FR)**
• **LEFEVRE, Fabrice**
**F-31190 Auterive (FR)**
• **NALIN, Renaud**
**F-31280 Dremil-Lafage (FR)**
• **REDZINIAK, Gérard**
**F-92160 Antony (FR)**

(74) Mandataire: **Gallois, Valérie et al**
**Cabinet BECKER & ASSOCIES**
**25, rue Louis Le Grand**
**75002 Paris (FR)**

(56) Documents cités:
**EP-A1- 1 384 482**　　**EP-A2- 1 917 956**
**WO-A1-2006/006757**　**GB-A- 896 940**
**KR-A- 20060 008 155**　**US-A1- 2002 119 952**
**US-A1- 2007 281 009**　**US-A1- 2008 020 997**

## Description

### Domaine de l'Invention

La présente invention est relative aux domaines cosmétiques et thérapeutiques.

### Contexte de l'Invention

La D-glucosamine (glucosamine) est un sucre aminé qui est un important précurseur de la synthèse biochimique des protéines et des lipides glycosylés. Elle est communément utilisée dans le traitement de l'arthrose, bien que son acceptation en tant que thérapie varie. On pense que la glucosamine favorise la formation et la réparation du cartilage. En 2001, un essai clinique de trois ans en double aveugle impliquant 212 patients souffrant d'arthrose (Reginster et al, 2001, Lancet, 357, 251-6) a montré une amélioration des symptômes de 20 à 25 % dans le groupe prenant de la glucosamine alors que le groupe placebo a montré une légère détérioration. Un examen aux rayons X a montré une diminution sérieuse de l'espace articulaire du genou (signe de progression de la maladie) chez pour la moitié des patients prenant de la glucosamine.

La D-N-acétyl-glucosamine (N-acétyl-glucosamine) est un dérivé de glucosamine qui présente une meilleure stabilité. La glucosamine et la N-acétyl-glucosamine sont des métabolites clés de fonctions biochimiques ou sont présents dans des polymères structuraux humains et animaux. Parmi ces polymères, les glycosaminoglycanes sont de longs polysaccharides non-branchés consistant en une unité disaccharide répété. L'unité répétée est un hexose (6 atomes de carbone) ou un acide hexuronique, lié à une hexosamine (6 atomes de carbone comprenant un azote comme la N-acétyl-glucosamine). Des exemples de glycosaminoglycanes sont la chondroïtine sulfate (cartilage élastique, cartilage hyalin, os, derme, cornée), le dermatane sulfate (derme, tendon, ligament, cartilage fibreux), le kératane sulfate (cartilage, cornée), l'héparine / héparane sulfate (foie, poumon, aorte), et le hyaluronane (peau).

Les glycosaminoglycanes sont un composant important du tissu conjonctif et peuvent représenter jusqu'à 30 % de la matière organique. Les chaînes de glycosaminoglycanes peuvent être liées de manière covalente à une protéine pour former des protéoglycanes. Par leur densité en sous-unités de sucre et leurs charges attirant les ions négatifs, l'eau se colle aux glycosaminoglycanes, procurant ainsi aux tissus une résistance accrue à la pression. Des exemples d'utilisations des glycosaminoglycanes incluent l'héparine comme anti-coagulant, les chondroïtines que l'on trouve dans les tissus conjonctifs, le cartilage et les tendons, le hyaluronane qui est un composant de la structure extracellulaire de la peau et du lubrifiant du liquide synovial dans les articulations.

De par leurs fonctions mécaniques, structurales et physiologiques, les glycosaminoglycanes sont des polymères majeurs pour la vie et la régulation de leur métabolisme est un élément important de la santé humaine et animale. Ainsi, il existe un besoin constant de compositions permettant de moduler la production des glycosaminoglycanes.

De manière intéressante, la N-acétyl-glucosamine a été utilisée pour différentes applications dans le domaine de la santé et de la cosmétique pour sa capacité à stimuler la synthèse de hyaluronane. La N-acétyl-glucosamine est produite soit par hydrolyse acide de chitine contenue dans la carapace de crevettes ou de crabes, soit par fermentation (au moins pour ce qui est de la glucosamine qui peut ensuite être acétylée). Une formulation topique de la N-acétyl-glucosamine a été décrite dans la demande de brevet US008/0020997. EP1384482 décrit la combinaison de la N-acétyl-glucosamine et d'un rétinoïde ou de la provitamine A pour augmenter la production d'acide hyaluronique.

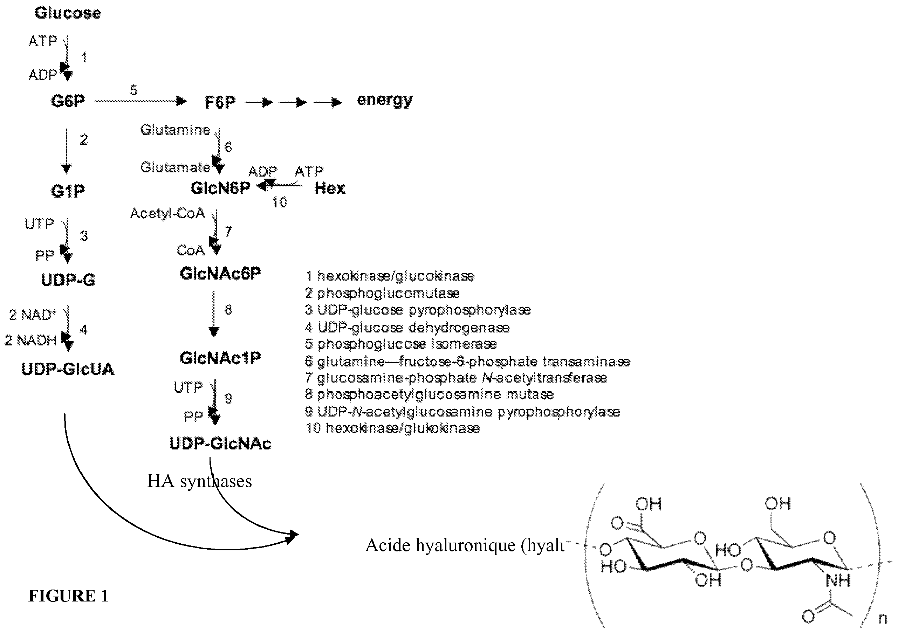

La N-acétyl-glucosamine-6-phosphate est une forme phosphorylée en C6 de la N-acétyl-glucosamine. Il a été démontré que la N-acétyl-glucosamine-6-phosphate est un intermédiaire dans la synthèse des glycosaminoglycanes tels que le hyaluronane. Dans cette voie spécifique, la N-acétyl-glucosamine-6-phosphate est isomérisée en N-acétyl-glucosamine-1-phosphate avant d'être activée sous forme d'UDP-N-acétyl-glucosamine et incorporée dans le polymère (voir Figure 1). La N-acétyl-glucosamine-6-phosphate est donc un métabolite intermédiaire qui est produit dans les cellules (composé endogène) et qui n'est pas synthétisé pour être excrété dans l'espace péricellulaire. Il semble de plus que la N-acétyl-glucosamine-6-phosphate ne soit pas produite par les cellules mammifères directement à partir de la N-acétyl-glucosamine.

Les sucres phosphorylés sont bien connus pour être des composés à haute énergie utilisés par les cellules à des carrefours de plusieurs voies métaboliques. La plupart de ces sucres phosphorylés sont des composés transitoires recyclés de manière permanente par les cellules, comme le glucose-6-phosphate ou le fructose-6-phosphate. Etant un métabolite intermédiaire dans les cellules, les sucres phosphorylés sont difficilement isolables dans des quantités suffisantes à des coûts raisonnables pour permettre de les tester. En outre, les sucres phosphorylés sont instables et ils sont rapidement hydrolysés par des phosphatases cellulaires et bactériennes en leurs portions sucrées non-phosphatées correspondantes, excluant ainsi leur utilisation directe.

Bien qu'un seul document GB 896940 mentionne en 1962 l'utilisation de la N-acétyl-glucosamine-6-phosphate parmi les dérivés de la N-acétyl-glucosamine pour favoriser la cicatrisation de plaies (sans fournir aucune donnée expérimentale avec ce composé), aucun document accessible au public ou à la communauté scientifique ne décrit, à notre connaissance, l'évaluation de la N-acétyl-glucosamine-6-phosphate pour ces potentiels effets thérapeutiques ou cosmétiques.

Ainsi, il reste un besoin constant de mettre au point des compositions cosmétiques et pharmaceutiques influençant la production des glycosaminoglycanes et leur domaine d'utilisation est très large.

### *Résumé de l'Invention*

Les inventeurs ont démontré des propriétés surprenantes et inattendues de la N-acétyl-glucosamine-6-phosphate en tant que composé exogène sur les cellules humaines, notamment dans la production des glycosaminoglycanes démontrant ainsi son utilité dans des compositions pharmaceutiques, vétérinaires et cosmétiques, seule ou en combinaison avec d'autres composés, en particulier un composé de la famille de la vitamine A, afin de promouvoir des effets bénéfiques sur la santé et l'esthétique. Ils ont également démontré un effet synergique pour la combinaison de la N-acétyl-glucosamine-6-phosphate avec un composé de la famille de la vitamine A.

L'objet de la présente invention est défini par les revendications et toute information non comprise dans les revendications est fournie à titre d'information.

La présente invention est donc relative à l'utilisation d'une composition cosmétique comprenant de la N-acétyl-glucosamine-6-phosphate ou un sel de celle-ci cosmétiquement acceptable et un composé de la famille de la vitamine A pour le traitement des peaux sèches ou pour le traitement ou la prévention du vieillissement de la peau ou des phanères, en particulier pour le traitement ou la prévention des rides, en particulier les rides profondes et/ou les ridules, pour l'amélioration de l'élasticité et/ou du tonus de la peau, pour le raffermissement de la peau, et/ou pour la régénération/renforcement de la jonction épidermo-dermique et/ou pour augmenter la prolifération des cellules cutanées (en particulier les kératinocytes et les fibroblastes) et/ou pour augmenter la synthèse de macromolécules structurales (en particulier le hyaluronane et/ou le collagène).

La présente invention est tout particulièrement relative à une composition cosmétique comprenant de la N-acétyl-glucosamine-6-phosphate ou un sel de celle-ci cosmétiquement acceptable et en outre un composé de la famille de la vitamine A sélectionné parmi le groupe consistant en le rétinol ou un ester de celui-ci, le rétinal ou rétinaldéhyde (cis ou trans), l'acide rétinoïque, l'isotrétinoïne, l'adapalène, la trétinoïne et le mélange de ceux-ci. Dans un mode de réalisation encore plus préféré, le composé de la famille de la vitamine A est le rétinol ou un ester de celui-ci. Cette composition peut être adaptée à une administration topique, orale ou injectable (en particulier adaptée à une injection intraépidermique et/ou intradermique et/ou transdermique et/ou sous-cutanée et/ou micro-injection).

La présente invention est également relative à une composition pharmaceutique ou vétérinaire comprenant de la N-acétyl-glucosamine-6-phosphate ou un sel de celle-ci pharmaceutiquement acceptable pour une utilisation dans le traitement du psoriasis, de la dermatite atopique, de l'eczéma, de troubles articulaires, de troubles liés au déficit de cartilage, et des épanchements de synovie, pour la viscosupplémentation, et dans des traitements accompagnant des actes chirurgicaux de l'oeil. Elle comprend en outre un composé de la famille de la vitamine A sélectionné parmi le groupe consistant en le rétinol ou un ester de celui-ci, le rétinal ou rétinaldéhyde (cis ou trans), l'acide rétinoïque, l'isotrétinoïne, l'adapalène, la trétinoïne et le mélange de ceux-ci. Dans un mode de réalisation encore plus préféré, le composé de la famille de la vitamine A est le rétinol ou un ester de celui-ci. Cette composition peut être adaptée à une administration topique, orale ou injectable (en particulier adaptée à une injection intraépidermique et/ou intradermique et/ou transdermique et/ou sous-cutanée et/ou intramusculaire et/ou intraveineuse et/ou micro-injection et/ou intra-articulaire).

La présente invention concerne également une composition pharmaceutique ou vétérinaire comprenant de la N-acétyl-glucosamine-6-phosphate ou un sel de celle-ci pharmaceutiquement acceptable et un composé de la famille de la vitamine A sélectionné parmi le groupe consistant en le rétinol ou un ester de celui-ci, le rétinal ou rétinaldéhyde (cis ou trans), l'acide rétinoïque, l'isotrétinoïne, l'adapalène, la trétinoïne et le mélange de ceux-ci. Dans un mode de réalisation encore plus préféré, le composé de la famille de la vitamine A est le rétinol ou un ester de celui-ci. Elle concerne une composition selon ce mode de réalisation pour la réparation et/ou la régénération de tissus endommagés.

La présente invention concerne l'utilisation de la N-acétyl-glucosamine-6-phosphate ou un sel de celle-ci pharmaceutiquement acceptable en combinaison avec un composé de la famille de la vitamine A, pour la préparation d'une composition pharmaceutique ou vétérinaire ou un médicament pour une utilisation dans le traitement du psoriasis, de la dermatite atopique, de l'eczéma, de troubles articulaires, de troubles liés au déficit de cartilage, et des épanchements de synovie, pour la viscosupplémentation, et dans les traitements accompagnant des actes chirurgicaux de l'oeil.

La présente invention est enfin relative à un dispositif comprenant une composition pharmaceutique, vétérinaire ou cosmétique selon la présente description, le dispositif étant adapté à une injection (de préférence intraépidermique et/ou intradermique et/ou transdermique et/ou sous-cutanée et/ou micro-injection et/ou dans l'espace articulaire) ou à une administration topique.

*Brève description des figures*

Figure 1 : Schéma de la voie de synthèse du hyaluronane dans les cellules mammifères.
Figure 2 : Analyse de la morphologie de la peau après traitement avec de la N-acétyl-glucosamine-6-phosphate
Figure 3 : Analyse de la production de glycosaminoglycanes dans la peau après traitement avec de la N-acétyl-glucosamine-6-phosphate
Figure 4 : Analyse de l'expression du récepteur CD44 dans la peau après traitement avec de la N-acétyl-glucosamine-6-phosphate

*Description détaillée de l'Invention*

Les inventeurs ont testé et identifié pour la première fois les effets de la N-acétyl-glucosamine-6-phosphate en tant que composé exogène (c'est-à-dire obtenu selon un procédé technique ne mettant en oeuvre aucun constituant du corps humain) sur les cellules humaines. Ils ont montré que, de manière surprenante, cette entité chimique peut être utilisée dans des compositions pharmaceutiques, dermatologiques, vétérinaires et cosmétiques, seule ou en combinaison avec d'autres composés, en particulier ceux de la famille de la vitamine A, pour promouvoir des effets bénéfiques sur la santé et l'esthétique par l'intermédiaire de différents mécanismes incluant :

- la stimulation de la synthèse cellulaire de macromolécules structurales du corps humain, incluant les glycosaminoglycanes comme le hyaluronane et les collagènes, en particulier du pro-collagène 1;
- l'augmentation de la production de CD44, récepteur à l'acide hyaluronique ;
- la stimulation de la division cellulaire, en particulier celle des kératinocytes et des fibroblastes; et,
- le renforcement de la jonction derme-épiderme.

Plus précisément, les inventeurs ont démontré la capacité de la N-acétyl-glucosamine-6-phosphate à augmenter la production de hyaluronane par les kératinocytes ou par les fibroblastes. En outre, ils ont démontré la capacité de la N-acétyl-glucosamine-6-phosphate à stimuler la division cellulaire des fibroblastes. La N-acétyl-glucosamine-6-phosphate permet aussi d'augmenter la synthèse de collagène, en particulier le pro-collagène 1, par les fibroblastes, notamment lorsqu'ils sont traités avec le surnageant des kératinocytes traités par la combinaison de la N-acétyl-glucosamine-6-phosphate avec les composés de la famille de la vitamine A. Enfin, sur des modèles de peau humaine, les inventeurs ont montré que le traitement par la N-acétyl-glucosamine-6-phosphate permet de stimuler la synthèse de glycosaminoglycanes, et ce de manière bien distribuée dans le derme entier, de stimuler la jonction derme-épiderme et de stimuler l'expression du récepteur CD44 dans le derme et l'épiderme.

En outre, les inventeurs ont mis en évidence des effets synergiques ou potentialisant de la combinaison de la N-acétyl-glucosamine-6-phosphate avec les composés de la famille de la vitamine A, en particulier le rétinol.

A contrario, la N-acétyl-glucosamine, seule ou en combinaison avec un composé de la famille de la vitamine A, montre des effets défavorables, ou ne montre pas d'effets, ou montre des effets qui ne sont pas comparables avec ceux de la N-acétyl-glucosamine-6-phosphate.

Le collagène représente également un groupe très important de polymères structuraux dans le corps humain. Le collagène est la protéine la plus abondante de l'organisme, représentant 25-35 % du contenu total en protéines de l'organisme. Il est produit par les cellules du tissu conjonctif. Le pro-collagène 1 est en particulier trouvé dans les tendons, la peau, la paroi des artères, l'endomysium des fibres musculaires striées, le fibrocartilage et la partie organique des os et des dents. Il est un constituant important de la matrice extracellulaire du derme, jouant un rôle dans l'intégrité de la peau. Le vieillissement de l'épiderme est dû à une atrophie de l'épiderme, conséquence du ralentissement de la prolifération des kératinocytes. Il entraîne en outre une diminution du collagène et donc un amincissement du derme, avec comme facteur aggravant l'exposition au soleil ou aux UV et le tabagisme. Le collagène et l'acide hyaluronique sont connus comme agents pour lutter contre les rides et ridules.

La jonction derme-épiderme est également cruciale pour la bonne nutrition de l'épiderme et l'élasticité de la peau.

C'est pourquoi la N-acétyl-glucosamine-6-phosphate, en combinaison avec un composé de la famille de la vitamine A, présente un intérêt certain dans une composition cosmétique pour prévenir ou diminuer les effets du vieillissement de la peau ou des phanères, en particulier des cheveux. Une telle composition est donc utile pour prévenir ou diminuer les rides, en particulier les rides profondes et/ou les ridules, pour améliorer l'élasticité cutanée, pour raffermir la peau. Dans un mode de réalisation où la composition de l'invention est injectée (en particulier micro-injection), elle est utile pour le comblement des rides et ridules et pour augmenter le volume des lèvres. Dans ce mode de réalisation, elle peut comprendre en outre ou être utilisée en combinaison avec des produits de comblement, notamment des produits résorbables comme l'acide hyaluronique, les graisses, le collagène ou les protéines ou non-résorbables comme les gels de polyacrylamide ou des silicones. Elle peut aussi être utilisée pour la régénération ou le renforcement de la jonction épidermo-dermique. Elle peut être utilisée pour la stimulation des cellules cutanées (notamment les mélanocytes, les kératinocytes et/ou les fibroblastes) par exemple en augmentant leur prolifération et/ou la migration cellulaire et/ou la synthèse des macromolécules structurales, notamment collagène et hyaluronane. Cette composition permet donc de maintenir et/ou de renforcer le derme et/ou l'épiderme, de maintenir et/ou de renforcer leur épaisseur, de maintenir et/ou de renforcer la tonicité et l'élasticité de la peau. Cette composition est adaptée pour les soins du corps et/ou du visage et/ou du cou et/ou des cheveux.

Dans un premier mode de réalisation particulier, la N-acétyl-glucosamine-6-phosphate et le composé de la famille de la vitamine A peuvent être les seuls principes ou agents actifs de la composition. Dans un autre mode de réalisation particulier, la composition comprend d'autres agents ou principes actifs. Des exemples de ces autres agents ou principes actifs sont détaillés plus loin. Par principe ou agent actif, on entend des composés qui présentent un effet biologique.

Dans cette application, la composition cosmétique peut être formulée pour être adaptée à une administration par voie orale, par voie topique sur la peau, le cuir chevelu ou les cheveux, ou par injection intraépidermique et/ou intradermique et/ou transdermique et/ou sous-cutanée et/ou micro-injection.

La composition cosmétique selon la présente invention pour une administration topique peut être sous la forme de solutions aqueuses, hydroalcooliques, d'émulsions (huile dans l'eau (H/E), eau dans l'huile (E/H), ou multiple (par exemple triple H/E/H ou E/H/E), de nanoémulsions, de gels aqueux ou de dispersions de phase grasse dans une phase aqueuse. Elle peut être formulée sous forme d'une crème plus ou moins fluide, d'un gel, d'un hydrogel, d'un produit filmogène (notamment à base d'acide hyaluronique de haut poids moléculaire qui a la propriété de capturer l'eau et de former un gel à l'étalement), d'une lotion, de masque, d'un lait, d'une huile, d'un onguent, d'une cire, d'une mousse, d'une pâte, d'un sérum, d'une pommade, d'un aérosol, d'un stick, d'un savon ou d'un shampoing.

La composition cosmétique adaptée à une administration topique peut être mise en oeuvre en combinaison avec des éléments mécaniques tels que des dispositifs à palper-rouler ayant une action mécanique et massante faisant pénétrer le produit et activant la circulation du produit ou des systèmes émetteurs d'ondes (lumière, basses fréquences, fréquences infrarouges, etc...) qui permettent d'activer la réponse de la peau ou d'augmenter directement l'effet du produit.

La composition cosmétique selon la présente invention pour une administration orale peut être sous la forme de cachet, pilule, gélule, sirop ou comprimé.

La composition cosmétique selon la présente invention pour une administration injectable est de préférence formulée sous une forme stérile. Elle peut aussi se présenter sous forme de poudre, le solvant étant ajouté extemporanément au moment de l'utilisation.

Les composants de la composition cosmétique ainsi que leurs proportions peuvent être aisément choisis par l'homme du métier sur la base de ses connaissances générales. Par exemple, la composition peut inclure, de manière non-exhaustive, de l'eau désionisée, du magnésium, du silicate d'aluminium, de la gomme de xanthane, du nylon-12, du sodium PCA, du propylène glycol, des oxydes de fer rouge, du talc, des oxydes de fer jaune, des oxydes de fer noir, du dioxyde de titane, du stéarate de glycérol, de l'acide stéarique, du DEA-cétyl phosphate, du méthylparabène, butylparabène, éthylparabène, propylparabène, isopropylparabène, isobutylparabène, du néopentanoate d'isostéaryle, du palmitate d'isopropyle, de l'éthylène/propylène/styrène, des copolymères, du copolymère de buthylène/propylène/styrène, du phénoxyéthanol, de l'acétate de tocophéryle, de la glycérine, du triéthylamine, de l'acide stéarique, du stéarate de propylène glycol, des huiles minérales, de l'urée de diazolidinyle, du poyisobutène hydrogéné, du palmitate d'octyle, du néopentanoate de tridécyle, du néopentanoate d'octyldodécyle, des parfums, du méthoxycinnamate d'octyle, de la benzophénone, du salicylate d'octyle, de l'isostéarate d'isopropyle, de l'actétate d'isoceteth-3 et des combinaisons de ceux-ci.

La composition cosmétique peut être également comprise dans un dispositif adapté au mode d'administration considéré.

De tels dispositifs peuvent être adaptés à une administration topique. On peut notamment citer les patchs, les pansements occlusifs, les mini-chambres d'occlusion, etc... Par patch, on entend tout type de patch connu de l'homme du métier, et notamment ceux qui génèrent un léger courant au niveau de la peau lorsqu'il est collé sur celle-ci permettant de favoriser la pénétration du produit dans celle-ci. Par mini-chambres d'occlusion, on entend un dispositif du type petite poche qui se colle à la peau, remplie de la composition cosmétique et qui permet de maintenir une quantité importante de cette composition au contact de la peau pour augmenter la quantité de produit qui va diffuser dans celle-ci.

D'autres dispositifs seront adaptés à une injection, en particulier adaptée à une injection intraépidermique et/ou intradermique et/ou transdermique et/ou sous-cutanée et/ou micro-injection. De tels dispositifs peuvent comprendre une aiguille, des micro-aiguilles ou un dispositif d'injection sans aiguille. De tels dispositifs sont bien connus en mésothérapie.

L'invention est relative à des kits comprenant la composition selon la présente invention et une seringue ou un dispositif injecteur.

Une composition comprenant la N-acétyl-glucosamine-6-phosphate, éventuellement en combinaison avec un composé de la famille de la vitamine A, peut également être utile pour la production de tissus humains *in vitro* ou *ex vivo,* notamment de la peau synthétique par exemple dans le contexte de l'ingénierie tissulaire. Ainsi, la présente invention est relative à une méthode pour produire un tissu humain, de préférence un tissu cutané ou conjonctif, comprenant la mise en contact des cellules du tissu ou amenées à former le tissu avec une composition comprenant de la N-acétyl-glucosamine-6-phosphate en combinaison avec un composé de la famille de la vitamine A. De préférence, les cellules comprennent des kératinocytes et/ou des fibroblastes.

De par l'impact de la N-acétyl-glucosamine-6-phosphate en combinaison avec un composé de la famille de la vitamine A sur la synthèse des glycosaminoglycanes, en particulier le hyaluronane, une composition pharmaceutique ou vétérinaire comprenant de tel(s) principe(s) actif(s) peut être utilisée dans le traitement ou la prévention des troubles articulaires ou ceux liés au déficit de cartilage, par exemple comme l'arthrite, l'arthrose, l'ostéoarthrite et l'ostéoarthrose, dans la stimulation de la synthèse de lubrifiant naturel, dans la viscosupplémentation et/ou dans le traitement de l'épanchement de synovie chez les humains et les animaux. Dans la présente demande, les animaux considérés sont de préférence des mammifères, par exemple des chevaux ou des animaux de compagnie comme les chiens et les chats. La présente invention est donc relative à l'utilisation de la N-acétyl-glucosamine-6-phosphate en combinaison avec un composé de la famille de la vitamine A pour la préparation d'une composition pharmaceutique ou vétérinaire destinée au traitement ou la prévention de troubles articulaires ou ceux liés au déficit de cartilage, par exemple comme l'arthrite, l'arthrose, l'ostéoarthrite et l'ostéoarthrose, pour la stimulation de la synthèse de lubrifiant naturel, pour la viscosupplémentation et/ou destinée au traitement de l'épanchement de synovie chez les humains et les animaux. Elle est aussi relative à une méthode de traitement de troubles articulaires ou ceux liés au déficit de cartilage, par exemple comme l'arthrite, l'arthrose, l'ostéoarthrite et l'ostéoarthrose, une méthode de traitement de l'épanchement de synovie, une méthode de viscosupplémentation ou de stimulation de la synthèse de lubrifiant naturel, chez un humain ou un animal le nécessitant, comprenant l'administration d'une dose efficace de la N-acétyl-glucosamine-6-phosphate en combinaison avec un composé de la famille de la vitamine A. Dans les applications envisagées dans ce mode de réalisation, N-acétyl-glucosamine-6-phosphate en combinaison avec un composé de la famille de la vitamine A peut être administrée ou formulée sous une forme adaptée à une administration topique, orale ou par injection. L'injection peut se faire par exemple par une injection dans l'espace articulaire, sous-cutanée, intramusculaire.

Par ailleurs, en considération des effets bénéfiques de la N-acétyl-glucosamine-6-phosphate sur la peau, une composition pharmaceutique, dermatologique ou vétérinaire comprenant de la N-acétyl-glucosamine-6-phosphate en combinaison avec un composé de la famille de la vitamine A peut être utilisée pour le traitement ou la prévention du psoriasis, de l'eczéma, de la dermatite atopique ou des peaux sèches.

En considération de l'effet exceptionnel de la combinaison de la N-acétyl-glucosamine-6-phosphate avec un composé de la famille de la vitamine A, notamment sur la production de hyaluronane par les kératinocytes et les fibroblastes, sur la division cellulaire des fibroblastes et sur la production de collagène par des fibroblastes, la présente demande considère tout particulièrement une composition pharmaceutique, dermatologique ou vétérinaire comprenant cette combinaison. Cette composition est utile dans la régénération des tissus endommagés. Ainsi, la présente invention est relative à l'utilisation de la N-acétyl-glucosamine-6-phosphate et d'un composé de la famille de la vitamine A pour la préparation d'une composition pharmaceutique, dermatologique ou vétérinaire destinée à la régénération des tissus endommagés ou à une méthode pour régénérer des tissus endommagés chez un sujet le nécessitant comprenant l'administration de quantité efficace de la N-acétyl-glucosamine-6-phosphate et d'un composé de la famille de la vitamine A.

En particulier, les compositions pharmaceutiques, dermatologiques ou vétérinaires de l'invention peuvent être utilisées dans un traitement accompagnant ou subséquent à des actes chirurgicaux de l'oeil (par exemple transplantation de cornée, glaucome, détachement de la rétine ou cataracte), des actes chirurgicaux ou dermatologiques de la peau (par exemple lifting, injections de produits, en particulier produits de comblement, peeling, blanchiment, dermabrasion, traitement de l'acnée), des actes chirurgicaux de l'abdomen.

Pour toutes les applications décrites dans la présente demande, il est à considérer que les formulations suivantes sont substituables les unes les autres :

-   composition pharmaceutique ou vétérinaire comprenant de la N-acétyl-glucosamine-6-phosphate ou un sel de celle-ci pharmaceutiquement acceptable en combinaison avec un composé de la famille de la vitamine A, pour une utilisation pour ...
-   utilisation de la N-acétyl-glucosamine-6-phosphate ou un sel de celle-ci pharmaceutiquement acceptable en combinaison avec un composé de la famille de la vitamine A, pour la préparation d'une composition pharmaceutique ou vétérinaire pour ...
-   produit comprenant de la N-acétyl-glucosamine-6-phosphate ou un sel de celle-ci pharmaceutiquement acceptable et un composé de la famille de la vitamine A comme produit de combinaison pour une utilisation simultanée, séparée ou étalée dans le temps pour ...
-   méthode pour ... chez un sujet le nécessitant comprenant l'administration d'une quatité efficace de la N-acétyl-glucosamine-6-phosphate pharmaceutiquement acceptable en combinaison avec un composé de la famille de la vitamine A.

Pour toutes ces applications, un mode de réalisation réside en l'utilisation combinée de la N-acétyl-glucosamine-6-phosphate et d'un composé de la famille de la vitamine A, en particulier du rétinol, ou en une composition comprenant une telle combinaison.

La composition pharmaceutique ou vétérinaire peut être formulée pour être adaptée à une administration par voie orale, par inhalation, topique (notamment sur la peau, le cuir chevelu, l'oeil ou une muqueuse, par exemple buccale, vaginale ou nasale), rectale, ou par injection parentérale, intraépidermique, intradermique, transdermique, sous-cutanée, intramusculaire, intraveineuse, intra-articulaire, intrasynoviale, intrasternale, intrathécale, intrahépatique, intralésioanle, ou intracraniale ou par des techniques d'infusion. De préférence, la composition pharmaceutique ou vétérinaire peut être formulée pour être adaptée à une administration par voie orale, par inhalation, topique (notamment sur la peau, le cuir chevelu, l'oeil ou une muqueuse, par exemple buccale, vaginal ou nasale), rectale, ou par injection intraépidermique, intradermique, transdermique, sous-cutanée, intra-articulaire, ou intrasynoviale. Par exemple, la composition pharmaceutique ou vétérinaire peut être formulée sous forme de cachets, de comprimés, de gélules, de comprimés, de sirops, de crèmes, de lotions, de gels. La composition peut comprendre des excipients dont une liste non exhaustive inclut le talc, le lactose, le stéarate de magnésium, le monostéarate de glycérol, du dioxyde de silicium colloïdale ou précipité, un polyvinyl pyrrolidone réticulé, du phosphate de calcium dibasique dihydraté, de la cellulose microcristalline, de l'amidon, du povidone, de la sodium carboxyméthyl cellulose, du polysorbate 80, de l'acide lactique, du carbomère, de l'alcool céthylique, du myristate d'isopropyle, du palmitate d'isopropyle, du glucose, du dextrose, du triéthylamine, de la glycérine, du fructose, du sucrose, des polymères et des nanostructures. La composition pharmaceutique ou vétérinaire peut être également comprise dans un dispositif adapté au mode d'administration considéré. Les formulations décrites pour la composition cosmétique peuvent s'appliquer à la composition pharmaceutique ou vétérinaire.

Dans le cas des formulations orales, et en particulier des comprimés, le support comprend souvent du lactose et de l'amidon. Des agents lubrifiants comme le stéarate de magnésium sont également ajoutés. Dans le cas des gélules, des diluants incluent le lactose et l'amidon. Pour les suspensions aqueuses, l'ingrédient actif est combiné avec des émulsifiants et des agents suspendants. Il est également possible d'incorporer des agents sucrants, colorants ou gustatifs.

Dans le cas de formulations rectales, comme les suppositoires, ces formulations peuvent être préparées en mélangeant le principe actif avec des excipients adaptés qui sont solides à température ambiante mais liquides à la température rectale. De tels matériaux incluent le beurre de cacao, de la cire d'abeille et des polyéthylèneglycols.

Pour une administration topique, la composition peut notamment être formulée sous forme d'onguent contenant le principe actif dissous ou suspendu dans des supports appropriés. De tels supports incluent de manière non-exhaustive des huiles minérales, de la vaseline liquide, de la vaseline blanche, du propylène glycol, du polyoxyéthylène, du polyoxypropylène, de la cire émulsifiante et de l'eau. Alternativement, elle peut être formulée sous forme de crème ou de lotion contenant le principe actif dissous ou suspendu dans des supports appropriés. De tels supports incluent de manière non-exhaustive des huiles minérales, du monostéarate de sorbitan, du polysorbate 60, de la cire d'esters de cétyle, de l'alcool cétéarylique, du 2-octyldodécanol, de l'alcool benzylique et de l'eau.

Pour une application ophtalmique, la composition peut être formulée sous forme d'une suspension micronisée dans une solution saline stérile isotonique à pH ajusté ou de préférence sous forme d'une solution dans une solution saline stérile isotonique à pH ajusté, sans ou avec conservateur anti-microbien tel que le chlorure de benzalkonium. Alternativement, la composition peut aussi être formulée sous forme d'onguent contenant une matière comme de la vaseline.

Dans les formes injectables, la composition peut être une suspension aqueuse ou oléagineuse. Ces suspensions peuvent être formulées selon les techniques bien connues de l'homme du métier en utilisant des agents mouillants ou dispersants et des agents de suspension. De préférence, elle sera sous forme de solution ou suspension stérile. Les solvants et supports acceptables incluent de manière non-exhaustive de l'eau, une solution de Ringer et une solution de chlorure de sodium isotonique. En outre, des huiles stériles fixes sont souvent utilisées dans le milieu de suspension ou le solvant. Dans ce but, une quelconque huile fixe mélangée peut être utilisée, comme des mono- et diglycérides. Des acides gras comme l'acide oléique et ses dérivés glycérides sont également utiles dans la préparation de compositions injectables, de même que des huiles naturelles pharmaceutiquement acceptables comme l'huile d'olive, l'huile de ricin, en particulier leurs versions polyoxyéthylénées. Ces solutions huileuses peuvent comprendre des agents de suspension ou de dilution comme la carboxyméthylcellulose pour la formulation d'émulsions et de suspensions. Des surfactants comme les Tweens, des agents émulsifiants, des agents augmentant la biodisponibilité peuvent aussi être inclus.

La N-acétyl-glucosamine-6-phosphate est un composé qui n'est pas disponible en grande quantité. Elle est donc préparée de préférence par le procédé décrit dans la demande de brevet WO08/142155 (en particulier dans l'exemple 1). Notamment, la N-acétyl-glucosamine de source marine (dérivée de la chitine de crevettes ou de crabes) est mélangée avec du phosphate inorganique sélectionné et l'enzyme phosphorylante adaptée dans un réacteur contenant de l'eau et des sels pendant plusieurs heures. Pendant cette réaction, l'enzyme greffe un phosphate sur la N-acétyl-glucosamine pour donner de la N-acétyl-glucosamine-6-phosphate. Ce composé peut alors être purifié à l'aide de plusieurs étapes de précipitation en utilisant différents alcools (tels que l'isopropanol et/ou l'éthanol). La solution aqueuse finale est purifiée sur une résine échangeuse d'ion pour enlever les ions restants. La N-acétyl-glucosamine-6-phosphate peut être concentrée dans l'eau et stérilisée par microfiltration (filtre de 0,2 $\mu$m). La pureté finale peut être évaluée par des méthodes analytiques. Cette méthode permet de préparer de la N-acétyl-glucosamine-6-phosphate qui peut être utilisée directement à partir de la solution aqueuse concentrée ou qui peut être séchée pour obtenir une poudre cristalline qui se dissout rapidement dans une quelconque formule aqueuse.

La N-acétyl-glucosamine-6-phosphate peut également être utilisée dans les compositions et les produits de la description sous forme de sels, en particulier un sel pharmaceutiquement ou cosmétiquement acceptable. Ainsi, de tels sels incluent notamment les sels d'addition acides, les sels d'addition basiques, les sels métalliques, et les sels d'ammonium ou d'ammonium alkylé, en particulier ceux pharmaceutiquement ou cosmétiquement acceptables. Les sels d'addition acides incluent les sels inorganiques et les sels organiques. Des exemples représentatifs d'acides inorganiques adaptés comprennent les acides chlorhydrique, bromhydrique, iodique, phosphorique, etc... Des exemples représentatifs d'acides organiques adaptés comprennent les acides acétique, formique, propionique, benzoïque, maléique, fumarique, succinique, tartrique, citrique, trichloroacétique, trifluoroacétique, etc... D'autres exemples de sels d'addition acides organiques ou inorganiques sont fournis dans J. Pharm Sci., 1977, 66, 2 et dans « Handbook of Pharmaceutical Salts : Properies, Selection and Use édité par P. Heinrich Stahl et Camille G. Wermuth, 2002. Des exemples de sels métalliques incluent les sels de lithium, sodium, potassium, ou magnésium, etc...Des exemples de sels d'ammonium et d'ammonium alkylés incluent les sels d'ammonium, de méthyl-ammonium, de diméthyl-ammonium, de diméthyl-ammonium, de triméthyl-ammonium, d'éthyl-ammonium, d'hydroxyéthyl-ammonium, de diéthyl-ammonium, de butyl-ammonium, tétraméthyl-ammonium, etc...Des exemples de bases organiques incluent la lysine, l'arginine, la guanidine, la diéthanolamineoline etc...

Les compositions de la présente invention, qu'elle soit cosmétique, pharmaceutique, dermatologique ou vétérinaire, peuvent comprendre un support physiologiquement acceptable, de préférence acceptable dans le domaine cosmétique, pharmaceutique, dermatologique ou vétérinaire, respectivement.

Par « composé de la famille de la vitamine A » est entendu dans la présente demande un composé sélectionné parmi le groupe consistant en le rétinol (aussi appelé vitamine A) ou un ester de celui-ci, le rétinal ou rétinaldéhyde (cis ou trans), l'acide rétinoïque (trans), le rétinaldéhyde, l'isotrétinoïne (acide cis-retinoïque), l'adapalène, la trétinoïne (acide rétinoïque, trans) et le mélange de ceux-ci. De préférence, la composé est sélectionné parmi le groupe consistant en le rétinol ou un ester de celui-ci, le rétinal ou rétinaldéhyde (cis ou trans), l'acide rétinoïque, le rétinaldéhyde, l'isotrétinoïne, la trétinoïne et le mélange de ceux-ci. Dans un mode de réalisation particulier, le composé de la famille de la vitamine A est le rétinol ou un ester de celui-ci.

La quantité de la N-acétyl-glucosamine-6-phosphate utilisée dans les compositions et produits de l'invention dépend de l'effet cosmétique ou thérapeutique et peut donc varier.

Dans un mode de réalisation particulier, une gamme de concentration de la N-acétyl-glucosamine-6-phosphate comprise entre environ 0,001 g/L ou g/kg et environ 1000 g/L ou g/kg de composition, et de préférence comprise entre environ 0,01 g/L ou g/kg et environ 100 g/L ou g/kg de composition, et de préférence encore comprise entre environ 0,1 g/L ou g/kg et environ 10 g/L ou g/kg de composition, semble adaptée, tout particulièrement pour une administration topique.

Dans un mode de réalisation plus préféré, le composé de la famille de la vitamine A est combiné à la N-acétyl-glucosamine-6-phosphate en une quantité synergique ou potentialisante.

Dans un mode de réalisation particulier, le composé de la famille de la vitamine A, en particulier le rétinol ou un ester de celui-ci, est présent dans la composition à une concentration comprise entre environ 3 $\mu$g/L ou $\mu$g/kg et environ 3 g/L ou g/kg de composition et de préférence entre environ 100 mg/L ou mg/kg et environ 1,5 g/L ou g/kg de composition.

Un des désavantages du rétinol est qu'il génère des effets secondaires indésirables, et notamment une irritation de la peau. La synergie ou l'effet potentialisant a été démontré à de faibles concentrations, en particulier des concentrations plus faibles que celles généralement utilisées en cosmétique et thérapie. Ainsi, dans un mode de réalisation préféré, le composé de la famille de la vitamine A est combiné à la N-acétyl-glucosamine-6-phosphate en une quantité non ou moins toxique et non ou moins irritante qui dépend de l'application finale de la composition. Ainsi, dans ce mode particulier de réalisation, le composé de la famille de la vitamine A, en particulier le rétinol ou un ester de celui-ci, est présent dans la composition à une concentration comprise entre environ 3 $\mu$g/L ou $\mu$g/kg et environ 50 mg/L ou mg/kg de composition et de préférence entre environ 3 $\mu$g/L ou $\mu$g/kg et environ 10 mg/L ou mg/kg de composition.

Les compositions et kits de la présente invention peuvent comprendre en outre d'autres composants, en particulier des composés présentant un effet. Notamment, ils peuvent comprendre par exemple des capteurs de radicaux libres, de préférence des polyphénols stabilisés tels que ceux décrits dans la demande de brevet US 2009/0233876. Par ailleurs, ils peuvent aussi comprendre des inhibiteurs de phosphatases pour éviter la dégradation de la N-acétyl-glucosamine-6-phosphate en N-acétyl-glucosamine, par exemple la cystéine. Ils peuvent aussi comprendre des inhibiteurs de hyaluronidase pour empêcher ou diminuer la dégradation de hyaluronane néo-synthétisé produit sous la stimulation de la N-acétyl-glucosamine-6-phosphate, par exemple du 6-palmitoyl-L-ascorbique acide, le 6 hexadécanotae ascorbique acide. Ils peuvent comprendre des activateurs des hyaluronane synthases de manière à accélérer la synthèse de hyaluronane stimulée par la N-acétyl-glucosamine-6-phosphate et avoir une action synergique avec celle-ci, par exemple des eicosanoides tels que la prostaglandine E2, ou des acides rétinoiques (trans). Ils peuvent comprendre des dérivés d'acide glucuronique, comme par exemple l'acide glucuronique, ses sels, esters et amides ou l'acide UDP-glucuronique, pour permettre d'accélérer la synthèse de hyaluronane stimulée par la N-acétyl-glucosamine-6-phosphate et avoir une action synergique avec celle-ci.

L'invention sera encore mieux comprise à la lecture des exemples qui suivent.

### *Exemples*

### EXEMPLE 1 : Stimulation de la production d'acide hyaluronique par des kératinocytes humains traités avec de la N-acétyl-glucosamine-6-phosphate

#### 1. Principe du test

Des kératinocytes humains normaux ont été mis en culture jusqu'à obtention de cellules confluentes. Les cellules ont été traitées pendant 48H avec les produits à évaluer. Les surnageants, contenant l'acide hyaluronique, ont été recueillis et le dosage effectué extemporanément. La quantification de l'acide hyaluronique a été effectué au moyen d'un test en microplaque de type ELISA.

Un dosage protéique a été réalisé pour chaque puits de la microplaque cellulaire.

#### 2. Produit testés

N-acétyl-glucosamine-6-phosphate (NAG6P) a été testée à 20, 10 et 5 mM

#### 3. Préparation des cellules

Les primocultures de kératinocytes humains normaux (KHN) ont été obtenues à partir de cellules isolées de prélèvements cutanés (chirurgie plastique).

Les KHN ont été ensemencés à la densité de 130 000 cellules/puits en milieu de culture spécifique KSFM, (Gibco® Invitrogen) dans une plaque 24 puits. La plaque a été placée dans une étuve humide (37°C, 5%$CO_2$) pendant 24H.

#### 4. Traitement des cellules

Les différents produits, aux concentrations respectives, ont été mis en présence des cellules (dilutions dans le milieu de culture KSFM, 600$\mu$l) pendant 48H. Des cellules ont été laissées sans traitement : condition témoin.

Chaque condition de traitement des cellules est évaluée sur 3 puits de kératinocytes humains normaux.

#### 5. Dosages de l'acide hyaluronique

Après 24H de traitement, un prélèvement intermédiaire de 220 $\mu$l des surnageants a été réalisé pour chaque puits. La plaque a été replacée à l'étuve 37°C, 5%$CO_2$.

Après 48H de traitement, les surnageants restants ($\approx$ 350$\mu$l) ont été prélevés et centrifugés.

Le dosage des surnageants cellulaires a été effectué selon la méthode décrite dans le kit Hyaluronan Enzyme-Linked Immunosorbent Assay kit (HA-ELISA, Echelon Biosciences Inc.). Il s'agit d'un dosage de type ELISA, révélation par spectrophotométrie effectuée à 405nm (Safire, Tecan).

Pour chaque puits cellulaire, des duplicata de mesures ont été effectués.

#### 6. Expression des résultats

Les mesures de densité optique DO obtenues pour chaque échantillon ont été reportées sur un graphique (courbe standard spécifique du kit). La concentration en acide hyaluronique a été déterminée en ng/ml.

7. Synthèse protéique

Il s'agissait de doser les protéines présentes dans les culots cellulaires correspondant aux surnageants pour lesquels le dosage d'acide hyaluronique a été réalisé.

Le dosage a été exprimé en $\mu$g de protéines par puits cellulaires.

Le principe de la méthode utilisée, Acide Bicinchoninique ou BCA, a été similaire à celui de la méthode de Lowry.

### 7.1. Principe

Les protéines réduisent le $Cu^{++}$ en $Cu^{+}$ de façon dose dépendante. L'acide bicinchoninique est un chromogène hautement spécifique des ions $Cu^{+}$, formant avec ces derniers un complexe coloré dont l'absorbance est mesurée à 550 nm.

### 7.2. Réactifs

Le dosage a été effectué à partir de deux solutions : l'acide bicinchoninique et le sulfate de cuivre. Lorsque ces deux réactifs sont mélangés, ils forment une solution verte réduite en violet en présence de $Cu^{+}$.

Ces réactifs sont regroupés dans le kit de dosage BCA-1 de Sigma. Une solution standard de protéines (BSA 1 mg/ml dans NaCl 0,15M) est également fournie.

### 7.3. Dosage des protéines

Après 48H de traitement, les surnageants des puits de la microplaque ont été prélevés pour le dosage d'acide hyaluronique. Ensuite, le tapis cellulaire repris dans une solution de PBS a été décollé par sonication puis dissous à l'aide d'une solution de NaOH 0,1 M. Le mélange acide bicinchoninique + sulfate de cuivre a été alors ajouté. La plaque a été placée à l'étuve humide (37°C, 5%$CO_2$) dans l'obscurité. Après 30 minutes, l'absorbance à 550 nm a été mesurée (Genios, Tecan).

En parallèle, une gamme étalon a été réalisée à partir d'une solution mère de BSA 1mg/ml (P-0914) : 0 à 100$\mu$g /puits.

Les mesures de densité optique des puits ont été converties en $\mu$g de protéines par puits de KHN grâce à la gamme.

### 7.4. Expression des résultats

Pour chaque puits cellulaires et chaque condition de traitement, on a obtenu un dosage quantitatif de la libération d'acide hyaluronique. Ce dosage a été rapporté à une quantité de cellules, exprimée en $\mu$g de protéines.

Le résultat final a donc été exprimé en ng/ml d'acide hyaluronique / $\mu$g de protéines.

Les valeurs quantitatives des puits traités avec la même concentration de produit ont été moyennées. Cette moyenne a été comparée à la moyenne des mesures obtenues pour les puits témoin (test t de Student - comparaison des moyennes - différence significative à 95% si $p<0,05^*$ et à 99% si $p<0,01^{**}$).

Un pourcentage de stimulation des produits a été déterminé en comparant les valeurs moyennes des conditions témoin et traitement :

$$\% \text{ Stimulation} = (\text{Traité - Témoin}) / \text{Témoin}$$

Les résultats sont exprimés dans les tableaux 1 et 2 (abréviation : NAG 6P : N-acétyl-glucosamine-6-phosphate).

*Tableau 1 : Dosage à 24h00 de l'acide hyaluronique produit par les kératinocytes*

| 24h00 - NAG6P | Acide Hyaluronique (ng/ml) | % Stimulation de la synthèse d'acide hyaluronique |
|---|---|---|
| Témoin | 789,5 $\pm$ 229 | |
| NAG 6P 10 mM | 1067,3 $\pm$ 111,9 | **35 %** * |
| * test Student : p<0,05 - résultat significatif à 95% | | |

*Tableau 2 : Dosage à 48h00 de l'acide hyaluronique produit par les kératinocytes*

| 48h00 - NAG6P | Acide Hyaluronique (ng/ml) | Protéines (µg/puits) | (ng/ml/µg prot.) | % Stimulation de la synthèse d'acide hyaluronique |
|---|---|---|---|---|
| Témoin | 1243,4 ± 232,3 | 17,9 ± 1,4 | 70,5 ± 18,4 | |
| NAG 6P 20 mM | 3076 ± 260,8 | 11,7 ± 1,9 | 269,6 ± 55,3 | **282% **\*\* |
| NAG 6P 10 mM | 2503,2 ± 552,8 | 15,3 ± 2,9 | 165,7 ± 32,4 | **135%** ** |
| NAG 6P 5 mM | 1817,9 ± 145,1 | 15,2 ± 2 | 122,1 ± 25,6 | **73%** * |
| * test Student : p<0,05 - résultat significatif à 95% <br> * * test Student : p<0,01 - résultat significatif à 99% | | | | |

Conclusions :

L'ajout de N-acétyl-glucosamine sur les kératinocytes humains normaux induit la stimulation de la production d'acide hyaluronique par ces cellules cutanées dès 24h00, de façon dose dépendante. Cet effet est confirmé et renforcé à 48h00 pour atteindre une augmentation du taux de production de +282% par rapport aux cellules n'ayant pas reçu de N-acétyl-glucosamine-6-phosphate.

La N-acétyl-glucosamine-6-phosphate est donc un excellent stimulateur de la production d'acide hyaluronique par les cellules de la peau. La NAG6P présente donc un intérêt pour stimuler le renouvellement de la matrice extracellulaire de la peau au niveau épidermique, afin de limiter la formation de rides et ridules, ou de promouvoir leur atténuation, et de favoriser et maintenir l'hydratation de la peau (grâce au fort pouvoir de capture de l'eau de l'acide hyaluronique).

**EXEMPLE 2 : Stimulation de la production d'acide hyaluronique par des kératinocytes humains traités avec de la N-acétyl-glucosamine-6-phosphate en synergie avec du propionate de vitamine A.**

Une expérience a été réalisée selon les conditions décrites dans l'exemple 1, en utilisant du propionate de vitamine A en synergie avec la N-acétyl-glucosamine-6-phoshate. Le propionate de vitamine A a été dissout dans du DMSO.

Pour ces conditions de test propionate de Vitamine A + / - NAG 6P, le pourcentage de stimulation a été exprimé par rapport au contrôle DMSO (dilution 1/10 000 correspondant à la quantité de DMSO effectivement présente) en remplacement du Témoin.

Les résultats sont exprimés dans les tableaux 3 et 4 (abréviation : NAG 6P : N-acétyl-glucosamine-6-phosphate).

*Tableau 3 : Dosage à 24h00 de l'acide hyaluronique produit par les kératinocytes*

| 24H - Synergie | Acide Hyaluronique (ng/ml) | % Stimulation de la synthèse d'acide hyaluronique |
|---|---|---|
| Témoin | 789,5 ± 229 | |
| NAG 6P 10 mM | 1067,3 ± 111,9 | **35%** * |
| Propionate Vit. A 1µM | 1056 ± 186,5 | **34%** * |
| NAG 6P 10 mM + Propionate Vit. A 1µM | 1569,6 ± 193 | **99%** ** |
| * test Student : p<0,05 - résultat significatif à 95% <br> * * test Student : p<0,01 - résultat significatif à 99% | | |

*Tableau 4: Dosage à 48h00 de l'acide hyaluronique produit par les kératinocytes*

| 48H - Synergie | Acide Hyaluronique (ng/ml) | Protéines (μg/puits) | (ng/ml/μg prot.) | % Stimulation de la synthèse d'acide hyaluronique |
|---|---|---|---|---|
| Témoin | 1243,4 ± 232,3 | 17,9 ± 1,4 | 70,5 ± 18,4 | / |
| NAG 6P 10 mM | 2503,2 ± 552,8 | 15,3 ± 2,9 | 165,7 ± 32,4 | **135% **\*\* |
| Contrôle DMSO[a] | 1028,9 ± 122,8 | 15,6 ± 3,3 | 66,9 ± 7,7 | / |
| Propionate Vit. A 1μM | 1944,8 ± 393,2 | 14,3 ± 1 | 137,1 ± 32,2 | **105% **\* |
| NAG 6P 10 mM + Propionate Vit. A 1μM | 3833,3 ± 216,3 | 13,5 ± 2 | 287,4 ± 35,7 | **330% **\*\* |
| [a] contrôle DMSO : dilution 1/10 000 correspondant à la quantité de DMSO présente dans le traitement avec le propionate de vitamine A et l'association NAG 10 mM + Propionate Vit. A 1μM. <br> \* test Student : p<0,05 - résultat significatif à 95% <br> \*\* test Student : p<0,01 - résultat significatif à 99% | | | | |

Conclusions:

De façon remarquable, il a été observé qu'en associant la N-acétyl-glucosamine-6-phosphate avec le propionate de vitamine A, un effet synergique sur la stimulation de la production d'acide hyaluronique par les kératinocytes est obtenu dès 24h00. De façon encore plus remarquable, cet effet est supérieur à la somme des effets de chaque composé pris individuellement (+99% d'augmentation du taux d'acide hyaluronique à 24h00 avec les deux composés versus +34% et +35% respectivement pour le propionate de vitamine A et la N-acétyl-glucosamine-6-phosphate seuls).

Cet effet synergique est confirmé et renforcé au bout de 48h00, puisque l'association de la N-acétyl-glucosamine-6-phosphate avec le propionate de vitamine A induit une augmentation de +330% de la production d'acide hyaluronique par les kératinocytes, contre +105% et +135% respectivement pour le propionate de vitamine A et la N-acétyl-glucosamine-6-phosphate seuls.

L'association de la N-acétyl-glucosamine-6-phosphate avec un dérivé de vitamine A améliore donc l'efficacité de la N-acétyl-glucosamine-6-phosphate sur la stimulation de la production d'acide hyaluronique par les cellules de la peau. La NAG6P en synergie avec le propionate de vitamine A présente donc un intérêt pour stimuler le renouvellement de la matrice extracellulaire de la peau au niveau épidermique, afin de limiter la formation de rides et ridules, ou de promouvoir leur atténuation, et de favoriser et maintenir l'hydratation de la peau (grâce au fort pouvoir de capture de l'eau de l'acide hyaluronique).

**EXEMPLE 3 : Stimulation indirecte (médiée par les kératinocytes) de la production d'acide hyaluronique par les fibroblastes humains normaux grâce à la N-acétyl-glucosamine-6-phosphate seule ou en association avec le propionate de vitamine A - comparaison avec la N-acétyl-glucosamine.**

1. Principe du test

Des fibroblastes dermiques humains normaux ont été mis en culture jusqu'à obtention de cellules confluentes. Les cellules ont été traitées pendant 48H avec des surnageants de kératinocytes humains normaux préalablement cultivés en présence des produits à évaluer. Les surnageants de fibroblastes, contenant l'acide hyaluronique, ont été recueillis et le dosage est effectué extemporanément.

La quantification de l'acide hyaluronique a été effectué au moyen d'un test en microplaque de type ELISA. Un dosage protéique a été réalisé pour chaque puits de la microplaque cellulaire.

2. Produits testés

* Produits N-acetyl-glucosamine (NAG) ou N-acétyl-glucosamine-6-phosphate (NAG6P) appliqués pendant 48H à 10 et 5 mM sur les kératinocytes humains normaux puis transfert du surnageant sur les fibroblastes humains normaux.
* Propionate de Vitamine A appliqué pendant 48H à 1 $\mu$M sur les kératinocytes humains normaux puis transfert du surnageant sur les fibroblastes humains normaux.
* Association NAG 10mM + Propionate de Vitamine A 1 $\mu$M appliquée pendant 48H sur les kératinocytes humains normaux puis transfert du surnageant sur les fibroblastes humains normaux.
* Association NAG6P 10mM + Propionate de Vitamine A 1 $\mu$M appliquée pendant 48H sur les kératinocytes humains normaux puis transfert du surnageant sur les fibroblastes humains normaux.

### 3. Préparation des cellules

Les primocultures de kératinocytes humains normaux (KHN) ont été obtenues à partir de cellules isolées de prélèvements cutanés (chirurgie plastique).
Les KHN ont été ensemencés à la densité de 130 000 cellules/puits en milieu de culture spécifique KSFM, (Gibco® Invitrogen) dans une plaque 24 puits. La plaque a été placée dans une étuve humide (37°C, 5%$CO_2$) pendant 24H.
Les primocultures de fibroblastes humains normaux (FHN) ont été obtenues à partir de cellules isolées de prélèvements cutanés (chirurgie plastique).
Les FHN ont été ensemencés à la densité de 90 000 cellules/puits en milieu de culture E-199 + 0,5% SVF, (Gibco® Invitrogen) dans une plaque 24 puits. La plaque a été placée dans une étuve humide (37°C, 5%$CO_2$) pendant 24H.
La mise en plaque des 2 types cellulaires KHN et FHN a été effectué à 48H d'intervalle.

### 4. Traitement des cellules

Les différents produits, aux concentrations respectives, ont été mis en présence des KHN (dilutions dans le milieu de culture KSFM, 600$\mu$l) pendant 48H. Des cellules ont été laissées sans traitement : condition témoin. 2 plaques cellulaires ont été utilisées pour évaluer séparément NAG et NAG en association au propionate de vitamine A d'une part puis NAG6P et NAG6P en association au propionate de vitamine A d'autre part.
Chaque condition de traitement des cellules est évaluée sur 3 puits de KHN.
Après 48H, une partie des surnageants cellulaires de KHN (300$\mu$l/puits) a été prélevée. On a procédé alors au transfert puits pour puits des surnageants cellulaires de KHN dans des surnageants cellulaires de FHN (300$\mu$l de E-199/puits - milieu frais sans SVF).
Ainsi on a obtenu un volume final de 600 $\mu$l/puits de traitement (contenu dans un milieu KSFM/E-199 50/50) qui reste appliqué 48 H sur les FHN.

### 5. Dosages de l'acide hyaluronique

Après 48H de traitement, les surnageants cellulaires de FHN ont été prélevés et centrifugés. Le dosage d'acide hyaluronique dans les surnageants cellulaires a été effectué selon la méthode décrite dans l'exemple 1. Pour chaque puits cellulaire, des duplicata de mesures sont effectués.

### 6. Expression des résultats

Les mesures de densité optique DO obtenues pour chaque échantillon ont été reportées sur un graphique (courbe standard spécifique du kit). La concentration en acide hyaluronique a été déterminée en ng/ml.

### 7. Synthèse protéique

Il s'agit de doser les protéines présentes dans les culots cellulaires correspondant aux surnageants pour lesquels le dosage d'acide hyaluronique a été réalisé. Le dosage a été réalisé selon la méthode décrite dans l'exemple 1, et le résultat a été exprimé en $\mu$g de protéines par puits cellulaires.
Pour chaque puits cellulaires et chaque condition de traitement, on a obtenu un dosage quantitatif de la libération d'acide hyaluronique. Ce dosage a été rapporté à une quantité de cellules, exprimée en $\mu$g de protéines.
Le résultat final a donc été exprimé en ng/ml d'acide hyaluronique/$\mu$g de protéines.
Les valeurs quantitatives des puits traités avec la même concentration de produit ont été moyennées. Cette moyenne a été comparée à la moyenne des mesures obtenues pour les puits témoin (test t de Student - comparaison des moyennes - différence significative à 95% si $p<0,05$* et à 99% si $p<0,01$**).

Un pourcentage de stimulation des produits a été déterminé en comparant les valeurs moyennes des conditions témoin et traitement :

$$\% \text{ Stimulation} = (\text{Traité} - \text{Témoin}) / \text{Témoin}$$

Pour les conditions propionate de Vitamine A + / - NAG ou NAG 6P (transférées), le pourcentage de stimulation a été exprimé par rapport au contrôle DMSO (dilution 1/10 000 correspondant à la quantité de DMSO effectivement présente) en remplacement du Témoin.

Les résultats présentés dans les tableaux 5 et 6 ci-après correspondent à la moyenne de 3 puits.

*Tableau 5 : Dosage à 48h00 de l'acide hyaluronique produit par les fibroblastes - effet de la NAG seule ou en association avec le propionate de vitamine A*

| 48H - NAG | Acide Hyaluronique (ng/ml) | Protéines ($\mu$g/puits) | (ng/ml/$\mu$g prot.) | % Stimulation de la synthèse d'acide hyaluronique |
|---|---|---|---|---|
| Témoin | $3559,3 \pm 374$ | $8,4 \pm 0,8$ | $429,9 \pm 78,4$ | / |
| NAG 10 mM<br>NAG 5 mM | $4056,1 \pm 315,5$<br>$4277,6 \pm 488$ | $15,7 \pm 0,6$<br>$12,2 \pm 2,7$ | $258,8 \pm 8,1$<br>$358,9 \pm 81,8$ | **- 40% \*\***<br>**-17%** |
| Contrôle DMSO[a] | $3001,7 \pm 354,6$ | $8 \pm 2,3$ | $387,3 \pm 73,4$ | / |
| Propionate Vit. A 1$\mu$M | $3645,6 \pm 667,8$ | $12 \pm 3,7$ | $316,6 \pm 68$ | **- 18%** |
| NAG 10 mM + Propionate Vit. A 1$\mu$M | $4439,4 \pm 445,9$ | $10 \pm 1,2$ | $443,4 \pm 15,2$ | **15%** |

[a] contrôle DMSO : dilution 1/10 000 correspondant à la quantité de DMSO présente dans le traitement avec le propionate de vitamine A et l'association NAG 10 mM + Propionate Vit. A 1$\mu$M.
\* test Student : p<0,05 - résultat significatif à 95%
\* \* test Student : p<0,01 - résultat significatif à 99%

*Tableau 6 : Dosage à 48h00 de l'acide hyaluronique produit par les fibroblastes - effet de la NAG6P seule ou en association avec le propionate de vitamine A*

| 48H - NAG 6P | Acide Hyaluronique (ng/ml) | Protéines ($\mu$g/puits) | (ng/ml/$\mu$g prot.) | % Stimulation de la synthèse d'acide hyaluronique |
|---|---|---|---|---|
| Témoin | $3351 \pm 287,4$ | $8,9 \pm 0,9$ | $375,9 \pm 13,5$ | / |
| NAG 6P 10 mM<br>NAG 6P 5 mM | $3507,6 \pm 213,3$<br>$3524,1 \pm 198,6$ | $9,2 \pm 0,3$<br>$8,8 \pm 0,5$ | $382,9 \pm 20,5$<br>$401,7 \pm 38,3$ | **2%**<br>**7%** |
| Contrôle DMSO[a] | $3058 \pm 552$ | $8,4 \pm 0,7$ | $364,3 \pm 54,2$ | / |
| Propionate Vit. A 1$\mu$M | $3358,8 \pm 615,3$ | $8,8 \pm 0,9$ | $389,2 \pm 108,3$ | **7%** |
| NAG 6P 10 mM + Propionate Vit. A 1$\mu$M | $4075,4 \pm 433,4$ | $7,9 \pm 0,2$ | $517,6 \pm 28,2$ | **42% \*\*** |

[a] contrôle DMSO : dilution 1/10 000 correspondant à la quantité de DMSO présente dans le traitement avec le propionate de vitamine A et l'association NAG6P 10 mM + Propionate Vit. A 1$\mu$M.
\* test Student : p<0,05 - résultat significatif à 95%
\* \* test Student : p<0,01 - résultat significatif à 99%

Conclusions :

Après avoir incubé pendant 48h00 des kératinocytes en présence de NAG seule ou de propionate de vitamine A seul, puis avoir transféré le surnageant de ces cellules sur des fibroblastes, il a été observé une diminution de la synthèse d'acide hyaluronique par ces fibroblastes (diminution significative à 10 mM de NAG). La même expérience réalisée en combinant la NAG en association avec le propionate de vitamine A conduit à une faible stimulation de 15% de la production d'acide hyaluronique par les fibroblastes ayant reçu le surnageant de culture des kératinocytes.

De façon surprenante, en incubant dans les mêmes conditions des kératinocytes en présence de NAG6P seule ou de propionate de vitamine A seul, puis avoir transféré le surnageant de ces cellules sur des fibroblastes, il a été observé une petite stimulation de synthèse d'acide hyaluronique par ces fibroblastes. La même expérience réalisée en combinant la NAG6P en association avec le propionate de vitamine A conduit à une stimulation importante de 42% de la production d'acide hyaluronique par les fibroblastes ayant reçu le surnageant de culture des kératinocytes.

Contrairement à la N-acétyl-glucosamine qui n'a révélé aucun effet, la N-acétyl-glucosamine-6-phosphate en association avec le propionate de vitamine A est capable de stimuler la production d'acide hyaluronique par les cellules dermiques par l'intermédiaire des kératinocytes. L'association N-acétyl-glucosamine-6-phosphate en association avec le propionate de vitamine A stimule donc la production d'acide hyaluronique au niveau de cellules située naturellement au niveau du derme.

La NAG6P en synergie avec le propionate de vitamine A présente donc un intérêt pour stimuler le renouvellement de la matrice extracellulaire de la peau au niveau dermique, afin de limiter la formation de rides et ridules, ou de promouvoir leur atténuation, et de favoriser et maintenir l'hydratation de la peau (grâce au fort pouvoir de capture de l'eau de l'acide hyaluronique) en profondeur.

**EXEMPLE 4 : Stimulation indirecte (médiée par les kératinocytes) de la production de pro-collagène 1 par les fibroblastes humains normaux grâce à la N-acétyl-glucosamine-6-phosphate seule ou en association avec le propionate de vitamine A - comparaison avec la N-acétyl-glucosamine.**

1. Principe

Cette expérience a été réalisée selon la même méthode que celle détaillée dans l'exemple 3. Les surnageants de fibroblastes, contenant le pro collagène I, sont recueillis et le dosage effectué extemporanément. La quantification du pro collagène I a été effectué au moyen d'un test en microplaque de type EIA.

2. Dosages du pro collagène I

Après 48H de traitement, les surnageants cellulaires de FHN ont été prélevés et centrifugés.

Le dosage de pro collagène I dans les surnageants cellulaires a été effectué selon la méthode décrite dans le kit Procollagen Type I C-peptide EIA kit (MK101, Takara). Il s'agit d'un dosage de type EIA, révélation par spectrophotométrie effectuée à 450nm (Safire, Tecan).

Pour 2 puits cellulaires, des duplicata de mesures ont été effectués tandis que le 3ème puits est évalué en simple dosage.

3. Expression des résultats

Les mesures de densité optique DO obtenues pour chaque échantillon ont été reportées sur un graphique (courbe standard spécifique du kit). La concentration en procollagène I a été déterminée en ng/ml.

Comme dans l'exemple 3, un dosage des protéines présentes dans les culots cellulaires correspondant aux surnageants pour lesquels le dosage de pro collagène I a été réalisé.

Le résultat final est donc exprimé en ng/ml de pro collagène I /$\mu$g de protéines.

Les valeurs quantitatives des puits traités avec la même concentration de produit ont été moyennées. Cette moyenne a été comparée à la moyenne des mesures obtenues pour les puits témoin (test t de Student - comparaison des moyennes - différence significative à 95% si $p<0,05^*$ et à 99% si $p<0,01^{**}$).

Un pourcentage de stimulation des produits a été déterminé en comparant les valeurs moyennes des conditions témoin et traitement :

$$\% \text{ Stimulation} = (\text{Traité - Témoin}) / \text{Témoin}$$

Pour les conditions Propionate de Vitamine A + / - NAG ou NAG 6P (transférées), le pourcentage de stimulation a été exprimé par rapport au contrôle DMSO (dilution 1/10 000 correspondant à la quantité de DMSO effectivement présente) en remplacement du Témoin.

Les résultats présentés dans les tableaux 7 et 8 ci-après correspondent à la moyenne de 3 puits.

*Tableau 7 : Dosage à 48h00 du* pro-collagène *1 produit par les fibroblastes - effet de la NAG seule ou en association avec le propionate de vitamine A*

| 48H - NAG | Pro collagène I (ng/ml) | Protéines ($\mu$g/puits) | (ng/ml/$\mu$g prot.) | % Stimulation |
|---|---|---|---|---|
| Témoin | 767,4 $\pm$ 94,3 | 8,4 $\pm$ 0,8 | 91,4 $\pm$ 20,6 | / |
| NAG 10 mM<br>NAG 5 mM | 700,9 $\pm$ 32,7<br>685,9 $\pm$ 38,6 | 15,7 $\pm$ 0,6<br>12,2 $\pm$ 2,7 | 44,9 $\pm$ 1,3<br>57,3 $\pm$ 10,5 | **- 51% \*\***<br>**- 37% \*** |
| Contrôle DMSO[a] | 611,7 $\pm$ 78,7 | 8 $\pm$ 2,3 | 75,7 $\pm$ 13,7 | / |
| Propionate Vit. A 1$\mu$M | 706,1 $\pm$ 73,8 | 12 $\pm$ 3,7 | 64,3 $\pm$ 20,1 | **15%** |
| NAG 10 mM +<br>Propionate Vit. A 1$\mu$M | 715 $\pm$ 26,3 | 10 $\pm$ 1,2 | 72,4 $\pm$ 9,6 | **-4%** |
| [a] contrôle DMSO : dilution 1/10 000 correspondant à la quantité de DMSO présente dans le traitement avec le propionate de vitamine A et l'association NAG 10 mM + Propionate Vit. A 1$\mu$M.<br>\* test Student : $p < 0,05$ - résultat significatif à 95%<br>\* \* test Student : $p < 0,01$ - résultat significatif à 99% | | | | |

*Tableau 8 : Dosage à 48h00 du* pro-collagène *1 produit par les fibroblastes - effet de la NAG6P seule ou en association avec le propionate de vitamine A*

| 48H - NAG 6P | Pro collagène I (ng/ml) | Protéines ($\mu$g/puits) | (ng/ml/$\mu$g prot.) | % Stimulation |
|---|---|---|---|---|
| Témoin | 675,5 $\pm$ 81,8 | 8,9 $\pm$ 0,9 | 73,2 $\pm$ 4,4 | / |
| NAG 6P 10 mM<br>NAG 6P 5 mM | 677,7 $\pm$ 64,6<br>653,4 $\pm$ 36,6 | 9,2 $\pm$ 0,3<br>8,8 $\pm$ 0,5 | 72,7 $\pm$ 4,4<br>74,6 $\pm$ 5,3 | **- 1%**<br>**2%** |
| Contrôle DMSO[a] | 672,4 $\pm$ 67,5 | 8,4 $\pm$ 0,7 | 78,0 $\pm$ 2,0 | / |
| Propionate Vit. A 1$\mu$M | 704,4 $\pm$ 38,6 | 8,8 $\pm$ 0,9 | 81,9 $\pm$ 13,0 | **5%** |
| NAG 6P 10 mM +<br>Propionate Vit. A 1$\mu$M | 744,7 $\pm$ 74,9 | 7,9 $\pm$ 0,2 | 94,7 $\pm$ 3,5 | **21% \*\*** |
| [a] contrôle DMSO : dilution 1/10 000 correspondant à la quantité de DMSO présente dans le traitement avec le propionate de vitamine A et l'association NAG 10 mM + Propionate Vit. A 1$\mu$M.<br>\* test Student : $p < 0,05$ - résultat significatif à 95%<br>\* \* test Student : $p < 0,01$ - résultat significatif à 99% | | | | |

Conclusions :

Après avoir incubé pendant 48h00 des kératinocytes en présence de NAG seule ou de propionate de vitamine A seul, puis avoir transféré le surnageant de ces cellules sur des fibroblastes, il a été observé une diminution de la synthèse de pro-collagène 1 par ces fibroblastes (diminution significative à 10 mM de NAG). La même expérience réalisée en combinant la NAG en association avec le propionate de vitamine A semble induire une diminution de la synthèse de pro-collagène 1 par ces fibroblastes.

De façon surprenante, en incubant dans les mêmes conditions des kératinocytes en présence de NAG6P en association avec du propionate de vitamine A, puis avoir transféré le surnageant de ces cellules sur des fibroblastes, il a été observé une stimulation de synthèse du pro-collagène 1 par ces fibroblastes. Cet effet de stimulation obtenu en combinant la NAG6P avec le propionate de vitamine A est supérieur à la somme des effets de ces composés pris individuellement.

Contrairement à la N-acétyl-glucosamine qui n'a révélé aucun effet, la N-acétyl-glucosamine-6-phosphate en association avec le propionate de vitamine A est capable de stimuler la production de pro-collagène 1 par les cellules dermiques par l'intermédiaire des kératinocytes. L'association N-acétyl-glucosamine-6-phosphate en association avec le propionate de vitamine A stimule donc la production de pro-collagène 1 au niveau de cellules naturellement présentes dans le derme.

La NAG6P est donc capable d'induire à travers les kératinocytes la production de pro-collagène 1 par les fibroblastes en synergie avec le propionate de vitamine A, et cette association présente donc un intérêt pour stimuler le renouvellement de la matrice extracellulaire de la peau afin de limiter la formation de rides et ridules, ou de promouvoir leur atténuation, et de favoriser et maintenir l'hydratation de la peau (grâce au fort pouvoir de capture de l'eau de l'acide hyaluronique) en profondeur.

**EXEMPLE 5 : Stimulation indirecte (médiée par les kératinocytes) de la prolifération de fibroblastes humains normaux grâce à la N-acétyl-glucosamine seule ou en association avec le propionate de vitamine A.**

Il s'agit d'évaluer la prolifération de fibroblastes humains normaux (primocultures dermiques) après traitement pendant 48H par des surnageants de Kératinocytes Humains transférés.

Le test « Cell Prolifération ELISA, BrdU » permet de quantifier la prolifération cellulaire, par mesure de l'incorporation de bromodéoxyuridine (BrdU) pendant l'étape de synthèse d'ADN suivie d'une détection en luminescence. C'est une méthode sensible et non radioactive (alternative à l'incorporation de [$H^3$] thymidine).

1. Principe

Les cellules ont éét mises en présence du traitement pour un temps défini (étuve 37°C, 5%$CO_2$). Ensuite l'analogue pyrimidine 5-bromo-2'-deoxyuridine (BrdU) a été ajouté aux cellules et celles-ci ont été remises en incubation (étuve 37°C, 5%$CO_2$) pour un temps minimal de 2H. Pendant cette période le BrdU est incorporé à l'ADN des cellules proliférantes à la place de la thymidine.

Le milieu de culture a été retiré, puis les cellules ont été fixées et l'ADN dénaturé dans le même temps (accessibilité de l'anticorps). Un anticorps anti BrdU-POD (peroxydase) a été ajouté. Celui-ci se fixe au BrdU incorporé dans l'ADN nouvellement synthétisé. Le complexe immunologique a alors été détecté par ajout du substrat luminol puis lecture en luminescence (Genios, Tecan).

2. Produits à tester

N-acétyl-glucosamine-6-phosphate (NAG6P) appliquée pendant 48H à 10 et 5 mM sur les kératinocytes humains normaux puis transfert du surnageant sur les fibroblastes humains normaux.

Propionate de Vitamine A appliqué pendant 48H à 1 µM sur les kératinocytes humains normaux puis transfert du surnageant sur les fibroblastes humains normaux.

Association NAG6P 10mM + Propionate de Vitamine A 1 µM appliquée pendant 48H sur les kératinocytes humains normaux puis transfert du surnageant sur les fibroblastes humains normaux.

3. Préparation des cellules

Les primocultures de kératinocytes humains normaux (KHN) ont été obtenues à partir de cellules isolées de prélèvements cutanés (chirurgie plastique).

Les KHN ont été ensemencés à la densité de 130 000 cellules/puits en milieu de culture spécifique KSFM, (Gibco® Invitrogen) dans une plaque 24 puits. La plaque a été placée dans une étuve humide (37°C, 5%$CO_2$) pendant 24H.

Les primocultures de fibroblastes humains normaux (FHN) ont été obtenues à partir de cellules isolées de prélèvements cutanés (chirurgie plastique).

Les FHN ont été ensemencés à la densité de 5 000 cellules/puits en milieu de culture E-199 (Gibco® Invitrogen) dans une plaque 96 puits blanche spécifique luminescence (BD Falcon 353947). La plaque a été placée dans une étuve humide (37°C, 5%$CO_2$) pendant 24H.

La mise en plaque des 2 types cellulaires KHN et FHN a été effectuée à 48H d'intervalle.

4. Traitement des cellules

Les différents produits, aux concentrations respectives, ont été mis en présence des KHN (dilutions dans le milieu de culture KSFM, 600µl) pendant 48H. Des cellules ont été laissées sans traitement : condition témoin. Chaque condition de traitement des cellules a été évaluée sur 3 puits de KHN.

Après 48H, une partie des surnageants cellulaires de KHN a été prélevée. Chaque surnageant prélevé (2 fois 100$\mu$l) issu d'un puits de la plaque de KHN (24 puits) a été transféré dans 2 puits de la plaque de FHN contenant déjà 100$\mu$l de E-199/puits.

Ainsi on a obtenu un volume final de 200 $\mu$l/puits de traitement (contenu dans un milieu KSFM/E-199 50/50) qui est resté appliqué 48 H sur les FHN.

5. Mesure de la prolifération

La quantification de la prolifération cellulaire a été efectuée selon la méthode décrite dans le kit « Cell Proliféra-ration ELISA, BrdU » (Roche Applied Science n°11669915001). Il s'agit d'un dosage de type immunologique, révélation en luminescence (Génios, Tecan).

6. Expression des résultats

Les mesures de luminescence (RLU/s) des 6 puits traités avec la même condition ont été moyennées. Cette moyenne a été comparée à la moyenne des mesures obtenues pour les 6 puits témoin (test t de Student - comparaison des moyennes - différence significative à 95% si $p<0,05*$ et à 99% si $p<0,01**$).

La prolifération des cellules traitées a été exprimé en pourcentage par rapport au témoin (cellules non traitées) de 100% (DO traité / DO témoin x 100).

Pour les conditions Propionate de Vitamine A + / - NAG 6P (transférées), le pourcentage de prolifération a été exprimé par rapport au contrôle DMSO (dilution 1/10 000 correspondant à la quantité de DMSO effectivement présente) en remplacement du Témoin.

Les résultats présentés dans le tableau 9 ci-après correspondent à la moyenne de 6 puits ayant reçus le transfert de 6 surnageants de KHN.

*Tableau 9 : Dosage à 48h00 de la prolifération cellulaire des fibroblastes - effet de la NAG6P seule ou en Association avec le propionate de vitamine A*

|  | RLU | % Prolifération |
|---|---|---|
| Témoin | 4121 $\pm$ 205 | / |
| NAG 6P 10 mM | 4903 $\pm$ 377 | **119% ** |
| NAG 6P 5 mM | 4985 $\pm$ 184 | **121% ** |
|  |  |  |
| Contrôle DMSO* | 4240 $\pm$ 187 | **100%** |
| Propionate Vit. A 1$\mu$M | 4640 $\pm$ 267 | **109% ** |
| NAG 6P 10 mM + Propionate Vit. A 1$\mu$M | 5693 $\pm$ 391 | **134% ** |

a contrôle DMSO : dilution 1/10 000 correspondant à la quantité de DMSO présente dans le traitement avec le propionate de vitamine A et l'association NAG 10 mM + Propionate Vit. A 1$\mu$M.
* test Student : $p<0,05$ - résultat significatif à 95%
* * test Student : $p<0,01$ - résultat significatif à 99%

Conclusions :

Après avoir incubé pendant 48h00 des kératinocytes en présence de NAG6P seule, puis avoir transféré le surnageant de ces cellules sur des fibroblastes, il a été observé de façon surprenante une augmentation significative de la prolifération des fibroblastes. De façon encore plus surprenante, cette augmentation n'est pas liée à la dose de NAG6P utilisée lors de la pré-incubation des kératinocytes, indiquant que ce n'est probablement pas le NAG6P qui pourrait agir directement sur la prolifération des fibroblastes. Le NAG6P induit donc très certainement une réponse des kératinocytes qui, sous l'effet de ce composé, produisent une ou des molécules (probablement de l'acide hyaluronique de différente taille) dans leur surnageant, qui elle(s)-même(s) vien(nen)t activer la prolifération des fibroblastes.

De façon remarquable, la combinaison de la NAG6P avec le propionate de vitamine A montre une stimulation plus forte de la prolifération des fibroblastes que chacun de ces produits testés séparément.

La NAG6P est donc capable d'induire à travers les kératinocytes la division des fibroblastes, seule ou en synergie avec le propionate de vitamine A, et présente donc un intérêt pour stimuler le renouvellement cellulaire de la peau.

**EXEMPLE 6 : Test des effets de la N-acétyl-glucosamine-6-phosphate sur peau humaine**

Lors de cette étude, l'effet de la N-acétyl-glucosamine a été évalué sur de la peau humaine maintenue en survie. Cet effet a été comparé à celui d'un crème commerciale 'RetinOX + Jour' contenant du rétinol (0,02 à 0,05%). Les effets suivants ont été plus particulièrement évalués au cours de cette étude :

- la morphologie générale de la peau après coloration au trichrome de Masson,
- la teneur en glycosaminoglycanes (GAGs) acides après coloration au bleu alcian,
- le taux d'expression du récepteur CD44 de l'acide hyaluronique après un immunomarquage de ce récepteur.

1. Produit testé

Produit testé : N-acétyl-glucosamine-6-phosphate (NAG6P) à 1% (P1) ; 0,1% (P2) et 0,01 % (P3) p/v.

2. Modèle biologique

A partir d'une plastie abdominale de femme de type caucasien de 36 ans (P712AB36), 33 explants (d'un diamètre d'environ 10 mm) ont été préparés et mis en survie en milieu spécifique de survie des explants cutanés. Les explants ont été répartis en 11 lots de 3 explants:

| Lot | Nb explants | Traitement | Prélèvement | Evaluations |
|---|---|---|---|---|
| T J0 | 3 | Aucun | J0 | Morphologie GAG CD44 |
| T J6 | 3 | Aucun | J6 | GAG CD44 |
| R J6 | 3 | Crème RetinOx + jour | J6 | GAG CD44 |
| P1 J6 | 3 | Actif NAG6P 1% | J6 | GAG CD44 |
| P2 J6 | 3 | Actif NAG6P 0,1% | J6 | GAG CD44 |
| P3 J6 | 3 | Actif NAG6P 0,01% | J6 | GAG CD44 |
| T J10 | 3 | Aucun | J10 | Morphologie GAG CD44 |
| R J10 | 3 | Crème RetinOx + jour | J10 | Morphologie GAG CD44 |
| P1 J10 | 3 | Actif NAG6P 1% | J10 | Morphologie GAG CD44 |
| P2 J10 | 3 | Actif NAG6P 0,1% | J10 | Morphologie GAG CD44 |

(suite)

| Lot | Nb explants | Traitement | Prélèvement | Evaluations |
|---|---|---|---|---|
| P3 J10 | 3 | Actif NAG6P 0,01% | J10 | Morphologie GAG CD44 |

### 3. Traitement

Aux jours J0, J2, J3, J6 et J8, la crème RetinOx+ jour a été appliquée sur les explants, à raison de 1 mg/cm$^2$.

A J0, J2, J3, J6 et J8, 30 $\mu$l des produits P1, P2 et P3 ont été déposés sur des disques de papier filtre appliqué sur les explants.

Les témoins ne reçoivent aucun traitement.

### 4. Prélèvements

A J0, les 3 explants du lot T0 ont été prélevés et coupés en deux. Une moitié a été fixée au Bouin ordinaire et l'autre moitié congelée à -80°C.

A J6 et à J10, 3 explants de chaque lot ont été prélevés et traités de la même manière.

### 5. Histologie

Après 48 heures de fixation dans le Bouin ordinaire, les prélèvements ont été déshydratés et imprégnés en paraffine à l'aide d'un automate de déshydratation Leica 1020. Ils ont été mis en bloc selon le mode opératoire MO-H-153 à l'aide d'une station d'enrobage Leica EG 1160. Des coupes de 5 $\mu$m ont été réalisées selon le mode opératoire MO-H-173 à l'aide d'un microtome type Minot, Leica RM 2125 et collées sur des lames de verre histologiques Superfrost®.

#### *5.1 Examen de la morphologie générale*

L'observation de la morphologie générale a été effectuée sur des coupes en paraffine après coloration au trichrome de Masson, variante de Goldner selon le mode opératoire MO-H-157.

#### *5.2 Visualisation des GAG acides*

Les GAG acides sont visualisés après coloration au bleu alcian. Ils sont observés dans tout le derme papillaire.

Dans cette zone, les GAGs recherchés apparaissent en bleu et sont des GAG acides. Une analyse d'image permet de quantifier l'intensité de la coloration.

#### *5.3 Immunomarquage du CD44*

Le CD44 a été marqué sur coupes congelées avec un anti-corps monoclonal anti-CD44, (Invitrogen MHC D4400), fait sur souris, au 1/100ème pendant une heure à température ambiant avec un système amplificateur biotine/streptavidine révélé en FITC avec les noyaux contre colorés à l'iodure de propidium.

Résultats

#### *Morphologie générale*

Au jour J0 :

Le stratum corneum des explants était modérément épais, très modérément feuilleté, nettement kératinisé en surface et à sa base. L'épiderme présentait 5 à 6 assises cellulaires avec une bonne morphologie. Le relief de la jonction dermo-épidermique était modéré. Le derme papillaire présentait un collagène avec des fibres assez épaisses formant un réseau assez dense. Il était bien cellularisé.

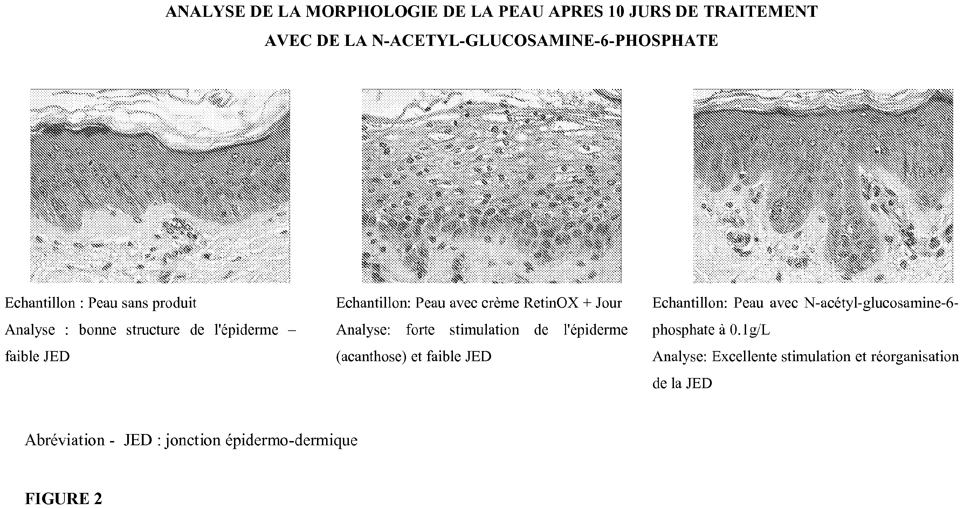

Au jour J10 (voir figure 2) :

La structure épidermique et dermique des explants témoins était légèrement altérée, avec une nette kératinisation à la base du stratum corneum et une légère parakératose. La spongiose était faible au niveau des couches basales.

L'application de la référence RetinOx+ jour, a induit une très nette réaction de type rétinoïque sur les explants avec une très nette acanthose épidermique (5 à 6 assises cellulaires/19 à 20). Le derme papillaire était dense.

L'application du produit NAG6P sur les explants a entraîné de nettes modifications de la morphologie épidermique. Le derme papillaire était assez dense. En particulier l'application du produit NAG6P à 0,01% (P3) a entraîné des évolutions de la morphologie générale et une nette réorganisation / renforcement de la jonction épidermo-dermique des explants. Le derme papillaire était plus ou moins dense.

**Visualisation / quantification des GAGs acides**

*Analyse visuelle*

Au jour J0 :

Les GAGs acides sont modérés sur l'ensemble du derme papillaire. Ils sont nets dans les fibroblastes.

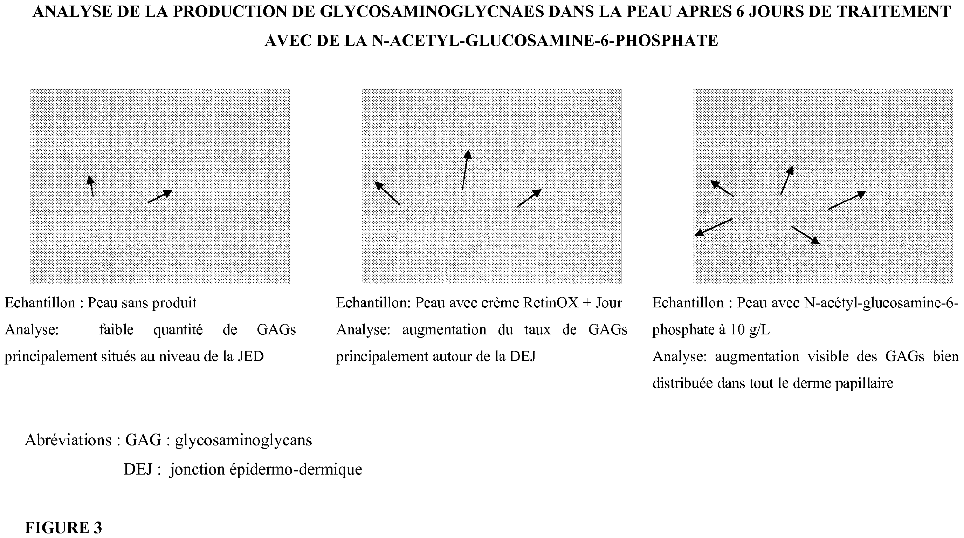

Au jour J6 (voir figure 3) :

Sur les explants non traités, ils ont été légèrement plus exprimés que ceux observés à T0.

Avec la crème RétinOx+ jour, ils ont été modérés et assez denses le long de la jonction épidermo-dermique (JDE) ainsi que dans le reste du derme papillaire.

Avec le produit NAG6P, ils ont été modérés le long de la JDE et présents dans tout le derme papillaire, et ce de façon dose dépendante : les GAGs acides ont été plus denses à 1% de NAG6P qu'à 0.01%.

Au jour J10 :

Sur les explants non traités, les GAGs ont été proches de ceux observés à J6.

Avec la référence RétinOx+ jour, ils ont été nets et assez denses le long de la JDE ainsi que dans le reste du derme papillaire.

Avec le produit NAG6P, l'effet observé à J6 se confirme : les GAGs acides ont été modérés le long de la JDE et présents dans tout le derme papillaire, et ce de façon dose dépendante : les GAGs acides ont été plus denses à 1% de NAG6P qu'à 0.01%.

*Analyse quantitative*

La quantification des GAGs acides a été réalisée par analyse d'image. Les résultats de cette quantification sont exprimés dans le tableau 10 ci-après, en pourcentage de la surface analysée.

*Tableau 10 : Pourcentage de surface occupé par les GAGs acides dans le derme papillaire*

| % de surface analysée | J0 | | J10 | |
|---|---|---|---|---|
| | Moyenne | Ecart-type | Moyenne | Ecart-type |
| Explants Témoins | 37,9 | 11,6 | **11,4** | 5,7 |
| Explants traités avec crème RetinOX+Jour | / | / | **23,8** | 8,7 |
| Explants traités avec NAG6P 1% | / | / | **21,0** | 3,4 |
| Explants traités avec NAG6P 0.1% | / | / | **16,3** | 6,3 |
| Explants traités avec NAG6P 0.01% | / | / | **14,0** | 4,0 |

*Expression du récepteur CD44 de l'acide hyaluronique*

Au jour J0 :

L'expression du CD44 était nette et très régulière dans les explants.
Dans l'épiderme, le CD44 était plus ou moins ponctiforme, bien membranaire et péri cellulaire dans toutes les couches vivantes jusqu'à la granuleuse. Dans le derme papillaire, il était très net sur les fibroblastes et sur des fibres fines.

Au jour J6 :

Sur le lot non traité, l'expression du CD44 était diminuée aussi bien dans l'épiderme que dans le derme papillaire.
Sur le lot traité avec la crème RetiOx + Jour, le CD44 était modérément surexprimé.
Le CD44 était légèrement surexprimé sur le lot traité avec le produit NAG6P à 1% et à 0,1%. Cette surexpression était très modérée avec le produit NAG6P à 0,01%.

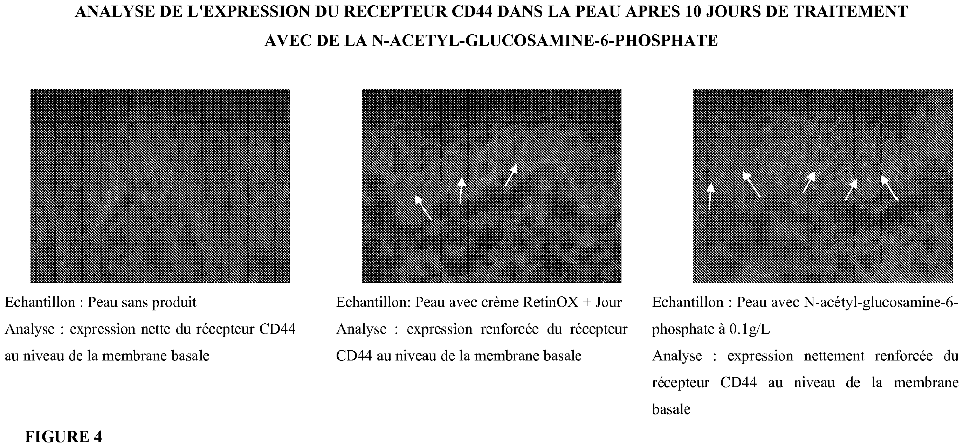

Au jour J10 (voir figure 4) :

Sur le lot non traité, l'expression du CD44 était légèrement surexprimée surtout dans l'épiderme sur les couches basales que dans le derme papillaire.
Sur le lot traité avec la crème RetiOx + Jour, le CD44 était modérément surexprimé.
Cette surexpression était très nette avec le produit NAG6P à 0,01% aussi bien dans l'épiderme que dans le derme papillaire.

Conclusions

Au cours de cette étude, il a été observé de façon remarquable que l'application de N-acétyl-glucosamine-6-phosphate sur de la peau humaine induit plusieurs phénomènes :

- une réorganisation observable de la structure épidermo-dermique avec un net renforcement de la jonction épiderme-derme, sans phénomène d'acanthose contrairement à la crème RetinOX + Jour ;
- une augmentation de la quantité de GAGs acide (donc principalement de l'acide hyaluronique qui est le principal GAG acide de la peau), non pas uniquement localisée au niveau de la jonction épidermo-dermique comme pour la crème RetinOX + Jour, mais bien répartie dans tout le derme papillaire ;
- une augmentation du taux de récepteur CD44 de l'acide hyaluronique, indiquant une activité de synthèse d'acide hyaluronique par la peau, et une activation des cellules cutanées.

De façon notable, ce composé seul agit de manière aussi voire plus efficace qu'une crème commerciale à base de rétinol (par exemple la N-acétyl-glucosamine-6-phosphate ne provoque pas de phénomène d'acanthose sur la peau contrairement à la crème commerciale).
La N-acétyl-glucosamine-6-phosphate présente donc un intérêt pour stimuler plusieurs phénomènes au niveau de la peau :

- la repulper à travers la stimulation de la synthèse des GAGs, composants de la matrice tri-dimensionnelle de soutient de la peau,
- renforcer les échanges de nutriments entre l'épiderme et le derme à travers une amélioration de la jonction épidermo-dermique,
- lisser la peau et la raffermir à travers l'amélioration de la jonction épidermo-dermique et la synthèse de GAGs
- promouvoir sa régénération à travers la stimulation du récepteur CD44.

**EXEMPLE 7 : Exemples de compositions pour le soin de la peau à base de N-acétyl-glucosamine-6-phosphate (NAG6P)**

| 6.1 Crème régénérante pour Peaux âgées (hors invention) | |
|---|---|
| *A-Phase aqueuse* | |
| Glycérine | 2.0 % |

(suite)

*A-Phase aqueuse*

| | |
|---|---|
| Hexylene glycol | 3.0 % |
| Gomme de xanthane | 0.5% |
| Conservateurs | qs |
| Carbomer | 0.35% |
| **NAG6P** | 0.1% |
| Eau | qsp.100 % |

*B-Phase Grasse*

| | |
|---|---|
| Squalane | 15% |
| Alcool cétylique | 2% |
| Arachidyl alcohol/behenyl alcohol/arachidylglucoside) | 1% |
| Stéarate de glycérol | 5 % |
| Eau | 1.5% |
| NaOH | 0.35% |
| Conservateur+parfum | qs |

## 6.2. Emulsion hydratante et émolliente

*A-Phase Grasse*

| | |
|---|---|
| Ceteareth-2 | 3,5 % |
| Ceteareth-21 | 2 à 4% |
| Huile de germe de blé | 3 % |
| Cyclomethicone | 7 % |
| Octyl Palmitate | 8% |

*B-Phase aqueuse*

| | |
|---|---|
| Eau | qsp.100 % |
| Glycérine | 7.0 % |
| Hexylene glycol | 3.0 % |
| **NAG6P** | 0.1% |
| Conservateurs | qs |

*C-Ingrédients ajoutés dans l'émulsion, à une température inférieure à 40°C.*

| | |
|---|---|
| Hyaluronate de sodium | 0,1 % |
| Eau | 5 % |
| Tocopherol | 0,05 % |
| Palmitate de vitamine A | 0,1 % |
| Phospholipides | 0,5 % |
| Céramides 3 | 0,1 % |
| Polyacrylamide & $C_{14-13}$ isoparaffine & laureth-7 | 2 à 3,5 % |

## 6.3 Lait-crème solaire pour peaux photovieillies (hors invention)

*A-Phase Grasse*

| | |
|---|---|
| Monostéreate de Glycerol | 2% |
| PEG-100 stéarate | 3% |
| C12-C15 alkyl benzoate | 10 % |
| Dimethicone | 5% |
| Acétate de tocophérol | 1 % |
| Octyl-triazone (Uvinul T150) | 1.5 % |
| Butyl Methoxy Dibenzoyl methane (Eusolex 9020) | 2.0 % |
| Acool cetostéarylique | 1% |

(suite)

*B-Phase aqueuse*

| | |
|---|---|
| Eau | qsp.100 % |
| Conservateurs | 0.6% |
| Glycérine | 7 % |
| Hexylene glycol | 3.0 % |
| Carbomer | 0.5% |
| Tetra sodium EDTA | 0,2 % |
| **NAG6P** | 0.1% |
| Hyaluronate de sodium HPM | 0,1 % |
| Eau | 5 % |
| NaOH | 0.5% |
| Conservateur+parfum | qs. |

### 6.4. Crème anti-rides post-injection « Filler » (comblement)

*A-Phase Grasse*

| | |
|---|---|
| Squalane | 5% |
| Alcool cetylique | 2% |
| Dimethicone | 5% |
| Octyl palmitate | 5% |

*B-Phase aqueuse*

| | |
|---|---|
| Butylène glycol | 0,5 - 4 % |
| Eau | qsp.100 % |
| **NAG6P** | 0.1% |
| Glycérine | 2.0 % |
| Hexylene glycol | 3.0 % |
| Gomme de xanthane | 0.5% |
| Conservateurs | qs |

*C-Ingrédients ajoutés dans l'émulsion, à une température inférieure à 40°C.*

| | |
|---|---|
| Acétate de tocophérol | 0,1 à 1 % |
| Pyridoxine | 0,01 à 0,05 % |
| Palmitate de vitamine A | 0,01 à 1% |
| d-Panthénol | 0,1 à 1 % |
| Acide citrique | 0,1 à 0,5 % |
| Gluconate de zinc | 0,1 à 1 % |
| Citrate trisodique | 1 à 2,5 % |
| Eau | 5% |

### 6.5 Lotion démaquillante pour peaux matures (hors invention)

| | |
|---|---|
| Polysorbate 20 | 1.0 % |
| Caprylyl/capryl glucoside (Oramix CG110) | 2.0 % |
| **NAG6P** | 0.1% |
| PEG-7 glyceryl cocoate | 0.5 % |
| Hexylene glycol | 4-5% |
| d-Panthénol | 0,1 % |
| Mannitol | 0.02 % |
| Conservateurs | qs |
| Eau | qsp.100 % |

**Revendications**

1. Utilisation non-thérapeutique d'une composition cosmétique comprenant de la N-acétyl-glucosamine-6-phosphate ou un sel de celle-ci cosmétiquement acceptable et un composé de la famille de la vitamine A sélectionné parmi le groupe consistant en le rétinol ou un ester de celui-ci, le rétinal ou rétinaldéhyde, l'acide rétinoïque, l'isotrétinoïne, l'adapalène, la trétinoïne, et le mélange de ceux-ci, pour le traitement des peaux sèches ou pour le traitement ou la prévention du vieillissement de la peau ou de ses phanères, pour l'amélioration de l'élasticité et/ou du tonus de la peau, pour le raffermissement de la peau, et/ou pour la régénération/renforcement de la jonction épidermo-dermique et/ou pour augmenter la prolifération des fibroblastes et/ou pour augmenter la synthèse de macromolécules structurales sélectionnées parmi le hyaluronane et/ou le pro-collagène 1.

2. Utilisation selon la revendication 1, pour le traitement ou la prévention des rides.

3. Utilisation selon la revendication 1, pour le traitement ou la prévention des rides profondes et/ou les ridules.

4. Utilisation selon l'une quelconque des revendications 1 à 3, **caractérisée en ce que** le composé de la famille de la vitamine A est le rétinol, ou un ester de celui-ci.

5. Utilisation selon l'une quelconque des revendications 1-3, dans laquelle la composition est adaptée pour une administration topique, orale ou injectable.

6. Utilisation selon la revendication 5, dans laquelle la composition est adaptée pour une administration topique.

7. Composition cosmétique comprenant de la N-acétyl-glucosamine-6-phosphate ou un sel de celle-ci cosmétiquement acceptable et un composé de la famille de la vitamine A sélectionné parmi le groupe consistant en le rétinol ou un ester de celui-ci, le rétinal ou rétinaldéhyde, l'acide rétinoïque, l'isotrétinoïne, l'adapalène, la trétinoïne, et le mélange de ceux-ci.

8. Composition pharmaceutique ou vétérinaire comprenant de la N-acétyl-glucosamine-6-phosphate ou un sel de celle-ci pharmaceutiquement acceptable et un composé de la famille de la vitamine A sélectionné parmi le groupe consistant en le rétinol ou un ester de celui-ci, le rétinal ou rétinaldéhyde, l'acide rétinoïque, l'isotrétinoïne, l'adapalène, la trétinoïne, et le mélange de ceux-ci

9. Composition selon la revendication 7 ou 8, comprenant le rétinol ou un ester de celui-ci.

10. Composition selon la revendication 8, pour une utilisation dans le traitement du psoriasis, de la dermatite atopique, de l'eczéma, de troubles articulaires, de troubles liés au déficit de cartilage, et des épanchements de synovie, pour la viscosupplémentation, ou dans les traitements accompagnant des actes chirurgicaux de l'oeil.

11. Composition selon l'une quelconque des revendications 7-10 adaptée à une administration topique, orale ou injectable.

12. Dispositif comprenant une composition selon l'une quelconque des revendications 7-8, le dispositif étant adapté à une injection.

13. Dispositif selon la revendication 12 étant adapté à une injection intraépidermique et/ou intradermique et/ou transdermique et/ou sous-cutanée et/ou micro-injection et/ou dans l'espace articulaire.

**Patentansprüche**

1. Nichttherapeutische Verwendung einer kosmetischen Zusammensetzung, umfassend N-Acetylglucosamin-6-phosphat oder ein kosmetisch verträgliches Salz davon und eine Verbindung der Vitamin A-Familie, ausgewählt aus der Gruppe, bestehend aus Retinol oder einem Ester davon, Retinal oder Retinaldehyd, Retinsäure, Isotretinoin, Adapalen, Tretinoin und dem Gemisch davon, für die Behandlung der trockenen Haut oder für die Behandlung oder Prävention der Alterung der Haut oder ihrer Hautanhangsgebilde, für die Verbesserung der Elastizität und/oder Spannkraft der Haut, für die Straffung der Haut und/oder für die Regeneration/Stärkung der Epidermis-Dermis-Verbindung und/oder für die Steigerung der Fibroblastenproliferation und/oder für die Steigerung der Synthese von strukturellen Makromolekülen, ausgewählt aus Hyaluronan und/oder Prokollagen I.

**2.** Verwendung gemäß Anspruch 1 für die Behandlung oder Prävention von Falten.

**3.** Verwendung gemäß Anspruch 1 für die Behandlung oder Prävention von tiefen Falten und/oder Fältchen.

**4.** Verwendung gemäß einem der Ansprüche 1 bis 3, **dadurch gekennzeichnet, dass** die Verbindung der Vitamin A-Familie Retinol oder ein Ester davon ist.

**5.** Verwendung gemäß einem der Ansprüche 1 bis 3, wobei die Zusammensetzung für eine topische, orale oder injizierbare Verabreichung geeignet ist.

**6.** Verwendung gemäß Anspruch 5, wobei die Zusammensetzung für eine topische Verabreichung geeignet ist.

**7.** Kosmetische Zusammensetzung, umfassend N-Acetylglucosamin-6-phosphat oder ein kosmetisch verträgliches Salz davon und eine Verbindung der Vitamin A-Familie, ausgewählt aus der Gruppe, bestehend aus Retinol oder einem Ester davon, Retinal oder Retinaldehyd, Retinsäure, Isotretinoin, Adapalen, Tretinoin und dem Gemisch davon.

**8.** Pharmazeutische oder tiermedizinische Zusammensetzung, umfassend N-Acetylglucosamin-6-phosphat oder ein kosmetisch verträgliches Salz davon und eine Verbindung der Vitamin A-Familie, ausgewählt aus der Gruppe, bestehend aus Retinol oder einem Ester davon, Retinal oder Retinaldehyd, Retinsäure, Isotretinoin, Adapalen, Tretinoin und dem Gemisch davon.

**9.** Zusammensetzung gemäß Anspruch 7 oder 8, umfassend Retinol oder einen Ester davon.

**10.** Zusammensetzung gemäß Anspruch 8 für eine Verwendung in der Behandlung von Psoriasis, atopischer Dermatitis, Ekzem, Gelenkbeschwerden, mit Knorpeldefizit verbundenen Beschwerden und Gelenkergüsse oder für die Viskosupplementation oder in den Behandlungen, die mit chirurgischen Eingriffen am Auge einhergehen.

**11.** Zusammensetzung gemäß einem der Ansprüche 7 bis 10, die für eine topische, orale oder injizierbare Verabreichung geeignet ist.

**12.** Vorrichtung, umfassend eine Zusammensetzung gemäß einem der Ansprüche 7 bis 8, wobei die Vorrichtung für eine Injektion geeignet ist.

**13.** Vorrichtung gemäß Anspruch 12, die für eine intraepidermale und/oder intradermale und/oder transdermale und/oder subkutane Injektion und/oder Mikroinjektion und/oder in den Gelenkraum geeignet ist.

**Claims**

**1.** Non-therapeutic use of a cosmetic composition comprising N-acetylglucosamine-6-phosphate or a cosmetically acceptable salt thereof and a compound of the vitamin A family selected from the group consisting of retinol or an ester thereof, retinal or retinaldehyde, retinoic acid, isotretinoin, adapalene, tretinoin, and the mixture thereof, to treat dry skin or to treat or prevent aging of the skin and appendages, to improve skin elasticity and/or tone, to tighten the skin, and/or to regenerate/strengthen the dermal-epidermal junction and/or to enhance fibroblasts proliferation and/or to enhance the synthesis of structural macromolecules selected among hyaluronan and/or pro-collagen 1.

**2.** Use according to claim 1, to treat or prevent wrinkles.

**3.** Use according to claim 1, to treat or prevent deep wrinkles and/or fine lines.

**4.** Use according to any one of claims 1 to 3, wherein the compound of the vitamin A family is retinol or an ester thereof.

**5.** Use according to any one of claims 1 to 3, wherein the composition is suitable for topical or oral administration or injection.

6. Use according to claim 5, wherein the composition is suitable for topical administration.

7. Cosmetic composition comprising N-acetylglucosamine-6-phosphate or a cosmetically acceptable salt thereof and a compound of the vitamin A family selected from the group consisting of retinol or an ester thereof, retinal or retinaldehyde, retinoic acid, isotretinoin, adapalene, tretinoin, and the mixture thereof.

8. Pharmaceutic or veterinary composition comprising N-acetylglucosamine-6-phosphate or a pharmaceutically acceptable salt thereof and a compound of the vitamin A family selected from the group consisting of retinol or an ester thereof, retinal or retinaldehyde, retinoic acid, isotretinoin, adapalene, tretinoin, and the mixture thereof.

9. Composition according to claim 7 or 8, comprising retinol or an ester thereof.

10. Composition according to claim 8, for use in the treatment of psoriasis, atopic dermatitis, eczema, joint disorders, disorders related to cartilage deficiency, and synovial effusions, for viscosupplementation, or for treatments associated with eye surgery.

11. Composition according to any one of claims 7-10 suitable for topical or oral administration or injection.

12. Device comprising a composition according to any one of claims 7 to 8, the device being suitable for injection.

13. Device according to claim 12 being suitable for intraepidermal and/or intradermal and/or transdermal and/or subcutaneous and/or micro-injection and/or intra-articular injection.

EP 2 552 397 B1

**Glucose**

ATP

ADP

1

**G6P** — 5 —→ **F6P** —→ —→ —→ energy

2

Glutamine

6

Glutamate

ADP   ATP

**G1P**

**GlcN6P** ◄——— **Hex**

10

UTP

3

Acetyl-CoA

7

PP

CoA

**UDP-G**

**GlcNAc6P**

2 NAD⁺

4

8

2 NADH

**GlcNAc1P**

**UDP-GlcUA**

UTP

9

PP

**UDP-GlcNAc**

1 hexokinase/glucokinase
2 phosphoglucomutase
3 UDP-glucose pyrophosphorylase
4 UDP-glucose dehydrogenase
5 phosphoglucose isomerase
6 glutamine—fructose-6-phosphate transaminase
7 glucosamine-phosphate N-acetyltransferase
8 phosphoacetylglucosamine mutase
9 UDP-N-acetylglucosamine pyrophosphorylase
10 hexokinase/glukokinase

HA synthases

Acide hyaluronique (hyalu

**FIGURE 1**

## ANALYSE DE LA MORPHOLOGIE DE LA PEAU APRES 10 JURS DE TRAITEMENT

## AVEC DE LA N-ACETYL-GLUCOSAMINE-6-PHOSPHATE

Echantillon : Peau sans produit

Analyse : bonne structure de l'épiderme – faible JED

Echantillon: Peau avec crème RetinOX + Jour

Analyse: forte stimulation de l'épiderme (acanthose) et faible JED

Echantillon: Peau avec N-acétyl-glucosamine-6-phosphate à 0.1g/L

Analyse: Excellente stimulation et réorganisation de la JED

Abréviation -  JED : jonction épidermo-dermique

**FIGURE 2**

EP 2 552 397 B1

# ANALYSE DE LA PRODUCTION DE GLYCOSAMINOGLYCNAES DANS LA PEAU APRES 6 JOURS DE TRAITEMENT AVEC DE LA N-ACETYL-GLUCOSAMINE-6-PHOSPHATE

Echantillon : Peau sans produit

Analyse: faible quantité de GAGs principalement situés au niveau de la JED

Echantillon: Peau avec crème RetinOX + Jour

Analyse: augmentation du taux de GAGs principalement autour de la DEJ

Echantillon : Peau avec N-acétyl-glucosamine-6-phosphate à 10 g/L

Analyse: augmentation visible des GAGs bien distribuée dans tout le derme papillaire

Abréviations : GAG : glycosaminoglycans

DEJ : jonction épidermo-dermique

**FIGURE 3**

EP 2 552 397 B1

**ANALYSE DE L'EXPRESSION DU RECEPTEUR CD44 DANS LA PEAU APRES 10 JOURS DE TRAITEMENT AVEC DE LA N-ACETYL-GLUCOSAMINE-6-PHOSPHATE**

Echantillon : Peau sans produit

Analyse : expression nette du récepteur CD44 au niveau de la membrane basale

Echantillon: Peau avec crème RetinOX + Jour

Analyse : expression renforcée du récepteur CD44 au niveau de la membrane basale

Echantillon : Peau avec N-acétyl-glucosamine-6-phosphate à 0.1g/L

Analyse : expression nettement renforcée du récepteur CD44 au niveau de la membrane basale

**FIGURE 4**

**EP 2 552 397 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 0080020997 B
- EP 1384482 A
- GB 896940 A
- WO 08142155 A
- US 20090233876 A

### Littérature non-brevet citée dans la description

- **REGINSTER et al.** *Lancet,* 2001, vol. 357, 251-6
- *J. Pharm Sci.,* 1977, vol. 66, 2
- Handbook of Pharmaceutical Salts : Properies, Selection and Use. 2002